# EUROPEAN PATENT SPECIFICATION

(11) **EP 1 888 064 B1**
(45) Date of publication and mention of the grant of the patent: **13.04.2011**
(21) Application number: 06772641.4
(22) Date of filing: 07.06.2006
(51) Int. Cl.: A61K 31/439, C07D 451/04, A61P 25/30

(54) **BENZTROPINAMINE ANALOGS AS DOPAMINE UPTAKE INHIBITORS**
BENZTROPINAMIN-ANALOGE ALS DOPAMIN-AUFNAHMEHEMMER
ANALOGUES DE LA BENZTROPINAMINE UTILISES COMME INHIBITEURS DE LA CAPTURE DE LA DOPAMINE

(30) Priority: 10.06.2005 US 689746 P
(43) Date of publication of application: 20.02.2008
(73) Proprietor: Government of the United States of America, Represented by the Secretary, Department of Health and Human Services, Rockville, Maryland 20852 (US)
(72) Inventor: NEWMAN, Amy Hauck, Phoenix, Maryland 21131 (US); GRUNDT, Peter, Baltimore, Maryland 21202 (US); KATZ, Jonathan L., Columbia, Maryland 21044 (US)
(74) Representative: Bohest AG
(86) International application number: PCT/US2006/022401
(87) International publication number: WO 2006/135715

(56) References cited:
- WO-A-2005/009440
- US-A- 2 678 317
- US-A- 5 792 775
- GRUNDT ET AL.: "N-8-Substituted benztropinamine analogs as selective dopamine transporter ligands" BIOORGANIC AND MEDICINAL CHEMISTRY LETTERS, vol. 15, 2005, pages 5419-5423, XP002402043
- DATABASE CA CHEMICAL ABSTRACTS SERVICE, COLUMBUS, OHIO, US; XP002402046 retrieved from STN Database accession no. 1985:505186
- DATABASE CA CHEMICAL ABSTRACTS SERVICE, COLUMBUS, OHIO, US; XP002402047 retrieved from STN Database accession no. 1994:164586

## Description

### BACKGROUND OF THE INVENTION

The present invention relates generally to a family of benztropinamina analogs, pharmaceutical compositions comprising them, and their use to treat mental disorders.

The significant public health and social problems resulting from cocaine abuse have stimulated research efforts directed toward elucidating the central mechanisms by which cocaine exerts its behavioral effects. The data from these studies suggest that the primary mechanism of the behavioral effects of cocaine appears to be related to the inhibition of dopamine uptake (see, Ritz, M. C., et al., Science, 237,1219-1223 (1987); and Kuhar et al., Trends Neurosci., 14, 299-301 (1991)) which results in an elevated concentration of dopamine in the synapse. As a consequence, considerable emphasis has been directed toward the dopamine transporter as a target for research and potential therapeutics for the treatment of cocaine abuse.

There have been several approaches to finding tropane analogs as potential medications for psychostimulant abuse and other mental disorders; see, for example, Singh, S., Chem, Rev., 100, 925-1024 (2000); Newman A. H. and Kulkami S. S., Med. Res. Rev., 22, 429-464 (2002); Newman, A. H., Med. Chem. Res., 8,1-11 (1998); Carroll, F. I. J. Med. Chem., 46,1775-1794 (2003).
US 2,678,317 A mentions N-benzhydryltropylamine, certain derivatives thereof which are substituted in the 4-position of one benzene ring, salts of the amine and the derivatives, such as quaternary ammonium salts, and a crystalline hemihydrate of the free amine. Exemplified are N-benzhydryltropylamine, N-benzhydryltropylamine perchlorate, N-benzhydryltropylamine oxalate, N-benzhydryltropylamine, N-benzhydryltropylamine dimethylsulfate, N-benzhydryltropylamine methiodide, N-benzhydryltropylamine hemihydrate, N-(4-chlorobenzhydryl)tropylamine oxalate, N-(4-chlorobenzhydryl)tropylamine dimethylsulfate and N-(4-methylbenzhydryl)tropylamine hydrochloride. It mentions antispasmodic, antihistamic and mydriatic properties of these compounds.
Acta Pharmaceutica Sinica 19(5), pp. 361-366, 1984, mentions 1,1-diphenyl-2-(3-tropanyl)ethane and discloses some 1,1-disubstituted 2-(3-tropanyl)ethanes having received CAS registry numbers 98043-11-9, 98043-99-0, 98043-12-0, 98043-00-6, 98043-02-8, 98043-01-7, 98043-04-0, 98043-14-2 and 98043-05-1. It discloses anticholinergic effect for its compounds.
Chinese Journal of Medicinal Chemistry 3(1), pp. 23-26, 1993, mentions 1,1-diphenyl-2-(3-tropanyl)ethane and discloses 1-(3-methylphenyl)-1-phenyl-2-(3-tropanyl)ethane and its hydrochloride. It discloses anticholinergic effect for its compounds.
US 5,792,775 A discloses 4'- and 4',4"-substituted 3-alpha-(diphenylmethoxy)tropane analogs, their inhibitive effect on dopamine uptake and their usefulness in treating cocaine abuse.
Some of the reported analogs have limited or poor solubility in aqueous systems or poor stability characteristics. Accordingly, there exists a need for tropane or benztropinamine analogs which have improved solubility and/or stability. The present invention provides such analogs.

### BRIEF SUMMARY OF THE INVENTION

The present invention provides a family of tropane analogs. More particularly, the present invention provides a family of benztropinamine analogs as defined in claim 1, and a family of benztropinamide analogs as defined in claim 17 for use in the treatment of a mental disorder.

The benztropinamine analogs of the present invention have a high affinity for the dopamine transporter and inhibit dopamine uptake, but they do not produce a significant stimulation of locomotor activity or cocaine-like subjective effects in a drug discrimination model. The benztropinamine analogs have one or more advantageous properties, e.g., improved water solubility, increased stability, and/or selectivity.

The benztropinamine analogs of the present invention find use as therapeutics, e.g., cocaine antagonists or cocaine substitutes, for the treatment of cocaine abuse. The present invention also provides a method of treating a patient for a mental disorder.

The benztropinamine analogs of the present invention find use as imaging probes for dopamine transporter/cocaine binding sites and as imaging probes for neurodegenerative disorders (e.g., Parkinson's disease). The present invention also provides a method of selectively imaging cocaine binding sites of the central nervous system of a human patient, the method comprising administering to the central nervous system of the patient a compound described above and detecting the binding of that compound to the central nervous system tissue.

Also disclosed is a method of detecting or monitoring parkinsonism in a patient, the method comprising administering to the patient a detectably labeled compound described above and detecting the binding of that compound to the central nervous system tissue. Using this method, one can diagnose and/or monitor Parkinson's disease, a neurological disorder characterized by the progressive degeneration of dopamine nerve terminals.

Other features and advantages of the invention and its preferred embodiments will become apparent from the detailed description which follows.

### BRIEF DESCRIPTION OF THE SEVERAL VIEWS OF THE DRAWING(S)

FIG. 1 illustrates a synthetic scheme to prepare benztropinamine analogs in accordance with embodiments of the present invention.

FIG. 2 graphically depicts cocaine-like locomotor stimulant effects of compound 2m and cocaine as a function of dose in mice.

FIG. 3 graphically depicts cocaine-like subjective effects of compound 2m ("V") and cocaine ("●") as a function of dose in mice. FIG. 3A depicts % drug responding as a function of dose; FIG. 3B depicts % control response rate as a function of dose.

### DETAILED DESCRIPTION OF THE INVENTION

In one aspect, the present invention provides compounds having the general Formula I: 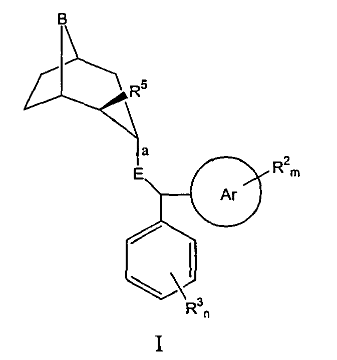 or a pharmaceutically acceptable salt or solvate thereof.

In Formula I, E is NR¹, S, or CH₂. Preferably, E is NR¹, R¹ is selected from the group consisting of hydrogen, C₁-C₁₂ alkyl, C₁-C₁₂ alkylamido C₁-C₁₂ alkyl, C₁-C₁₂ alkylamido C₅-C₂₀ aryl, C₂-C₁₂ alkylcarbonyloxy, C₂-C₁₂ alkoxyalkyl, C₁-C₁₂ hydroxyalkyl, C₃-C₁₂ alkylcarbonyloxyalkyl, C₅-C₂₀ aryl C₁-C₁₂ alkyl, C₅-C₂₀ aryloxy C₁-C₁₂ alkyl, cinnamyl, and C₂-C₁₂ alkylcarbonyl. Typically R¹ is hydrogen or C₁-C₁₂ alkyl. Preferably, R¹ is hydrogen or C₁-C₆ alkyl. More preferably, R¹ is hydrogen or C₁-C₃ alkyl, and even more preferably, R¹ is hydrogen or methyl.

The group Ar in Formula I is a C₅-C₂₀ monocyclic aryl group, a C₁₀-C₂₀ bicyclic aryl group, or a heteroaryl group having 2 to 10 carbon atoms and one or more heteroatoms selected from the group consisting of N, O, S, and any combination thereof. Typically Ar is selected from the group consisting of phenyl, naphthyl, biphenyl, pyridyl, bipyridyl, pyrimidyl, pyrrolyl, furanyl, thiophenyl, triazolyl, triazolopyrimidyl, thiadiazolyl, phosphole, diazaphosphole, quinoxalyl, benzofuranyl, benzopyrrolyl, morpholinyl, benzopyranyl, oxolyl, thiazolyl, purinyl, imidazolyl, indolyl, phosphindolyl (C₈H₆P-), pyrazolyl, and isoindolyl. Preferably, Ar is phenyl or naphthyl. More preferably, Ar is phenyl.

R² and R³ are each independently selected from the group consisting of hydrogen, halo, C₁-C₁₂ alkyl, C₁-C₁₂ alkoxy, nitro, cyanato, isocyanato, thiocyanato, amino, halo C₁-C₁₂ alkyl, hydroxyl, trihalo C₁-C₁₂ alkyl, and any combination thereof. Typically, R² and R³ are selected from the group consisting of hydrogen, halo, C₁-C₆ alkyl, C₁-C₆ alkoxy, nitro, cyanato, isocyanato, thiocyanato, amino, halo C₁-C₆ alkyl, hydroxyl, trihalo C₁-C₆ alkyl, and any combination thereof. Preferably R² is fluoro, chloro, or hydrogen and/or R³ is fluoro or chloro. More preferably, R² and R³ are fluoro.

The number of groups R² (m) and R³ (n) present in Formula I is m =1 to 5 and n = 1 to 3, respectively. In some embodiments, the phenyl and Ar rings each can have only one substituent such that m = n =1. In other embodiments, the phenyl ring can have one or two substituents (n is 1 or 2) while the Ar ring(s) can have 1 to 5 substituents (m = 1 to 5). R² and R³ can occupy any suitable position. Preferably R² and R³ do not occupy a position that is ortho to the methylene linkage, e.g., they occupy meta- and/or para-positions.

B is NR⁴, O, or CH₂. Preferably, B is NR⁴. R⁴ is selected from the group consisting of hydrogen, C₂-C₁₂ alkyl alkenyl, C₂-C₁₂ alkynyl, C₂-C₂₀ aryl C₁-C₁₂ alkyl, C₅-C₂₀ heteroaryl C₁-C₁₂ alkyl, C₁-C₁₂ alkylamino, heterocyclyl C₁-C₁₂ alkyl, C₁-C₁₂ alkylsulfonyl, C₂-C₁₂ alkylcarbonyl, (N(C₅-C₂₀ aryl)amido)C₁-C₁₂ alkyl, (N(C₁-C₁₂-alkyl)amido)C₁-C₁₂ alkyl, (N(C₅-C₂₀ aryl)amido)C₂-C₁₂ alkylcarbonyl, (N(C-₁C₁₂-alkyl)amido)C₂-C₁₂ alkylcarbonyl and, C₁-C₁₂ alkylamido C₅-C₂₀ aryl. Typically, R⁴ is selected from the group consisting of hydrogen, C₂-C₁₂ alkenyl, C₅-C₂₀ aryl-C₁-C₁₂ alkyl, C₁-C₁₂ alkylamido, heterocyclyl-C₂-C₁₂ alkyl C₁-C₁₂ alkyl, and (N(C₂-C₂₀-aryl)amido)C₁-C₁₂ alkyl. Preferably, R⁴ is selected from the group consisting of hydrogen, C₂-C₆ alkyl C₂-C₆ alkenyl, C₅-C₁₀ aryl-C₁-C₆ alkyl, C₁-C₆ alkylamino, heterocyclyl-C₁-C₆ alkyl, and (N(C₅-C₁₀-aryl)amido)C₁-C₆ alkyl. More preferably, R⁴ is selected from the group consisting of ethyl, propyl, butyl, allyl, phenylbutyl, 2-ethylamino, [2-(1*H*-indol-3-yl)-ethyl]-, and 3-[(N-phenyl)propionamido].

R⁵ is selected from the group consisting of hydrogen, hydroxyl, carboxyl, C₁-C₁₂ alkyl, C₁-C₁₂ alkoxy, C₂-C₁₂ alkylcarbonyl, C₂-C₁₂ carboxyalkyl, C₂-C₁₂ alkyloxycarbonyl, C₅-C₂₀ aryl, C₅-C₂₀ aryloxycarbonyl, C₅-C₂₀ aryl C₂-C₁₂ alkyloxycarbonyl, C₁-C₁₂ alkyl sulfonyl, C₁-C₁₂ hydroxyalkyle, formyl, C₂-C₁₂ formylalkyl, C₂-C₁₂ alkenyl, C₂-C₁₂ alkynyl, and any combination thereof. Typically R⁵ is selected from the group consisting of hydrogen, hydroxyl, carboxyl, C₁-C₆ alkyl, C₁-C₆ alkoxy, C₂-C₆ alkylcarbonyl, C₂-C₆ carboxyalkyl, C₂-C₆ alkyloxycarbonyl, C₅-C₁₀ aryl, C₅-C₁₀ aryloxycarbonyl, C₅-C₁₀ aryl C₂-C₆ alkyloxycarbonyl, C₁-C₆ alkyl sulfonyl, C₁-C₆ hydroxyalkyl, formyl, C₂-C₆ formylalkyl, C₂-C₆ alkenyl, C₂-C₆ alkynyl, and any combination thereof. Preferably R⁵ is H or C₂-C₁₂ alkylcarbonyloxy. More preferably R⁵ is hydrogen or C₂-C₆ alkylcarbonyloxy, for example, methylcarbonyloxy.

Bond "a" in Formula I can be of a, β, or α/β configuration. Preferably bond "a" is of a configuration.

Any of the groups R¹, R², R³, R⁴, and R⁵, other than hydrogen, halo, hydroxyl, nitro, cyanato, isocyanato, and thiocyanato can be further substituted with one or more substitutents selected from the group consisting of halo, hydroxyl, cyanato, thiocyanato, isocyanato, amino, C₁-C₁₂ alkyl, amido, nitro, and any combination thereof.

The term "independently selected" is used herein to indicate that the two R groups, i.e., R² and R³, can be identical or different (e.g., R² and R³ may both be methoxy), two or more R² groups may be identical or different, or two or more R³ groups may be identical or different.

The phrase "pharmaceutically acceptable salt" is intended to include nontoxic salts synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 18th ed., Mack Publishing Company, Easton, Pa., 1990, p. 1445, and Journal of Pharmaceutical Science, 66, 2-19 (1977).

Suitable bases include inorganic bases such as alkali and alkaline earth metal bases, e.g., those containing metallic cations such as sodium, potassium, magnesium, calcium and the like. Suitable acids include inorganic acids such as hydrochloric acid, hydrobromic acid, hydroiodic acid, sulfuric acid, phosphoric acid, and the like, and organic acids such as p-toluenesulfonic, methanesulfonic acid, benzenesulfonic acid, oxalic acid, p-bromophenylsulfonic acid, carbonic acid, succinic acid, citric acid, benzoic acid, acetic acid, fatty acids, long chain fatty acids, and the like. Examples of such pharmaceutically acceptable salts are the sulfate, pyrosulfate, bisulfate, sulfite, bisulfite, phosphate, monohydrogenphosphate, dihydrogenphosphate, metaphosphate, pyrophosphate, bromide, hydrobromide, iodide, acetate, propionate, caprate, caprylate, acrylate, ascorbate, formate, hydrochloride, dihydrochloride, isobutyrate, caproate, heptanoate, propiolate, glucuronate, glutamate, propionate, phenylpropionate, salicylate, oxalate, malonate, succinate, suberate, sebacate, fumarate, malate, maleate, hydroxymaleate, behenate, oleate, mandelate, nicotinate, isonicotinate, cinnamate, hippurate, nitrate, stearate, phthalate, terephthalate, butyne-1,4-dioate, butyne-1,4-dicarboxylate, hexyne-1,4-dicarboxylate, hexyne-1,6-dioate, benzoate, chlorobenzoate, methylbenzoate, hydroxybenzoate, methoxybenzoate, dinitrobenzoate, o-acetoxybenzoate, naphthalene-2-benzoate, p-toluenesulfonate, p-bromobenzenesulfonate, p-chlorobenzenesulfonate, xylenesulfonate, phenylacetate, trifluoroacetate, phenylpropionate, phenylbutyrate, citrate, lactate, hydroxybutyrate, glycolate, tartrate, hemi-tartrate, benzenesulfonate, methanesulfonate, ethanesulfonate, propanesulfonate, hydroxyethanesulfonate, 1-naphthalenesulfonate, 2-napththalenesulfonate, 1,5-naphthalenedisulfonate, and the like. Preferred pharmaceutically acceptable acid addition salts are those formed with mineral acids such as hydrochloric acid and hydrobromic acid, and those formed with organic acids such as maleic acid, oxalic acid, and methanesulfonic acid. Preferred pharmaceutically acceptable salts are hydrochloride, hydrobromide, oxalate, maleate, methanesulfonate, and hemi-tartrate. A particularly preferred pharmaceutically acceptable salt is hydrochloride. The compounds of the present invention are useful in the form of the free base or acid or in the form of a pharmaceutically acceptable salt thereof.

It should be recognized that the particular counterion forming a part of any salt of this invention is usually not of a critical nature, so long as the salt as a whole is pharmacologically acceptable and as long as the counterion does not contribute undesired qualities to the salt as a whole. It is further understood that the above salts may form solvates, or exist in a substantially uncomplexed form, such as the anhydrous form. As used herein, the term "solvate" refers to a molecular complex wherein the solvent molecule, such as the crystallizing solvent, is incorporated into the crystal lattice. When the solvent incorporated in the solvate is water, the molecular complex is called a hydrate. Pharmaceutically acceptable solvates include hydrates, alcoholates such as methanolates and ethanolates, acetonitrilates, and the like. These compounds can also exist in polymorphic forms.

Within the scope of Formula I, certain embodiments are preferred, for example, where bond "a" is of a configuration. For example, preferred compounds include those in which E is NR¹, R¹ is hydrogen or C₁-C₁₂ alkyl; m = n =1; R² and R³ are halo or hydrogen; B is NR⁴; R⁴ is selected from the group consisting of hydrogen, C₂-C₁₂ alkyl C₂-C₁₂ alkenyl, C₅-C₂₀ aryl-C₁-C₁₂ alkyl, C₁-C₁₂ alkylamino, heterocyclyl-C₁-C₁₂ alkyl, and (N(C₅-C₂₀-aryl)amido)C₁-C₁₂ alkyl; R⁵ is hydrogen; and Ar is phenyl; and bond "a" is of α configuration. In some embodiments, E is NR¹, R¹ is hydrogen or C₁-C₁₂ alkyl; m = n =1; R² and R³ are halo or hydrogen; B is NR⁴; R⁴ is selected from the group consisting of hydrogen, C₂-C₆ alkyl C₂-C₆ alkenyl, C₅-C₁₀ aryl-C₁-C₆ alkyl, C₁-C₆ alkylamino, heterocycyl-C₁-C₆ alkyl, and (N(C₅-C₁₀-aryl)amido)C₁-C₆ alkyl; R⁵ is hydrogen; Ar is phenyl; and bond "a" is of α configuration. Also preferred are compounds in which E is NR¹, R¹ is hydrogen or C₁-C₁₂ alkyl; m = n = 1; R² and R³ are halo or hydrogen; B is NR⁴; R⁴ is selected from the group consisting of n-butyl, allyl, phenylbutyl, 2-ethylamino, [2-(1*H*-indol-3-yl)-ethyl]-, and 3-[(N-phenyl)propionamido]; R⁵ is hydrogen; Ar is phenyl; and bond "a" is of α configuration.

In some embodiments of Formula I, E is NR¹, R¹ is hydrogen; m = n = 1; R² and R³ are halo or hydrogen; B is NR⁴; R⁴ is selected from the group consisting of hydrogen, C₂-C₁₂ alkyl C₂-C₁₂ alkenyl, C₅-C₂₀ aryl-C₁-C₁₂ alkyl, C₁-C₁₂ alkylamino, heterocyclyl-C₁-C₁₂ alkyl, and (N(C₅-C₂₀-aryl)amidoC₁-C₁₂ alkyl; R⁵ is hydrogen; Ar is phenyl; and bond "a" is of α configuration, specifically E is NR¹, R¹ is methyl; m = n = 1; R² and R³ are halo or hydrogen; B is NR⁴; R⁴ is selected from the group consisting of hydrogen, C₂-C₁₂ alkyl C₂-C₁₂ alkenyl, C₅-C₂₀ aryl-C₁-C₁₂ alkyl, C₁-C₁₂ alkylamino, heterocyclyl-C₁-C₁₂ alkyl, and (N(C₅-C₂₀-aryl)amido)C₁-C₁₂ alkyl; R⁵ is hydrogen; Ar is phenyl; and bond "a" is of α configuration; particularly E is NR¹, R¹ is hydrogen; m = n = 1; R² and R³ are chloro; B is NR⁴; R⁴ is selected from the group consisting of hydrogen, C₂-C₁₂ alkyl C₂-C₁₂ alkenyl, C₅-C₂₀ aryl-C₁-C₁₂ alkyl, C₁-C₁₂ alkylamino, heterocyclyl-C₁-C₁₂ alkyl, and (N(C₅-C₂₀-aryl)amido)C₁-C₁₂ alkyl; R⁵ is hydrogen; Ar is phenyl; and bond "a" is of α configuration; and more particularly E is NR¹, R¹ is hydrogen; m = n = 1; R² and R³ are fluoro; B is NR⁴; R⁴ is selected from the group consisting of hydrogen, C₂-C₁₂ alkyl C₂-C₁₂ alkenyl, C₅-C₂₀ aryl-C₁-C₁₂ alkyl, C₁-C₁₂ alkylamino, heterocyclyl-C₁-C₁₂ alkyl, and (N(C₅-C₁₁₂-aryl)amido)C₁-C₁₂ alkyl; R⁵ is hydrogen; Ar is phenyl; and bond "a" is of α configuration.

In other embodiments of Formula I, E is NR¹, R¹ is hydrogen or C₁-C₁₂ alkyl; m = n = 2; R² and R³ are halo or hydrogen; B is NR⁴; R⁴ is selected from the group consisting of hydrogen, C₂-C₁₂alkyl C₁-C₁₂ alkenyl, C₅-C₂₀ aryl-C₁-C₁₂ alkyl, C₁-C₁₂ alkylamino, heterocyclyl-C₁-C₁₂ alkyl, and (N(C₅-C₂₀-aryl)amido)C₁-C₁₂ alkyl; R⁵ is hydrogen; Ar is phenyl; and bond "a" is of a configuration; specifically E is NR¹, R¹ is hydrogen or C₁-C₁₂ alkyl; m = n = 2; R² and R³ are chloro or fluoro; B is NR⁴; R⁴ is selected from the group consisting of hydrogen, C₂-C₁₂ alkyl C₂-C₁₂ alkenyl, C₅-C₂₀ aryl-C₁-C₁₂ alkyl, C₁-C₁₂ alkylamino, heterocyclyl-C₁-C₁₂ alkyl, and (N(C₅-C₂₀-aryl)amido)C₁-C₁₂ alkyl; R⁵ is hydrogen; Ar is phenyl; and bond "a" is of a configuration; and more specifically E is NR¹, R¹ is hydrogen or methyl; m = n = 2; R² and R³ are chloro or fluoro; B is NR⁴; R⁴ is selected from the group consisting of hydrogen C₂-C₁₂ alkyl, C₂-C₁₂ alkenyl, C₅-C₂₀ aryl-C₁-C₁₂ alkyl, C₁-C₁₂ alkylamino, heterocyclyl-C₁-C₁₂ alkyl, and (N(C₅-C₂₀-axyl)amido)C₁-C₁₂ alkyl; R⁵ is hydrogen; Ar is phenyl; and bond "a" is of α configuration.

In other embodiments of Formula I, E is NR¹, R¹ is hydrogen or C₁-C₁₂ alkyl; m = n = 2; R² and R³ are halo or hydrogen; B is NR⁴; R⁴ is selected from the group consisting of hydrogen, C₂-C₆ alkyl, C₂-C₆ alkenyl, C₅-C₁₀ aryl-C₁-C₆ alkyl, C₁-C₆ alkylamino, heterocyclyl-C₁-C₆ alkyl, and (N(C₅-C₁₀-aryl)amido)C₁-C₆ alkyl; R⁵ is hydrogen; Ar is phenyl; and bond "a" is of α configuration; specifically E is NR¹, R¹ is hydrogen or C₁-C₁₂ alkyl; m = n = 2; R² and R³ are halo or hydrogen; B is NR⁴; R⁴ is selected from the group consisting of, n-butyl, allyl, phenylbutyl, 2-ethylamino, [2-(1*H*-indol-3-yl)-ethyl]-, and 3-[(N-phenyl)propionamido); R⁵ is hydrogen; Ar is phenyl; and bond "a" is of α configuration; and more specifically E is NR¹, R¹ is hydrogen or C₁-C₁₂ alkyl; m = n = 2; R² and R³ are chloro or fluoro; B is NR⁴; R⁴ is selected from the group consisting of n-butyl, allyl, phenylbutyl, 2-ethylamino, [2-(1*H*-indol-3-yl)-ethyl]-, and 3-[(N-phenyl)propionamido]; R⁵ is hydrogen; Ar is phenyl; and bond "a" is of a configuration.

It should be noted, however, that if bond "a" is of β configuration, Ar is phenyl, m = 1, R² is hydrogen, B is NCH₂CH₃, E is NH, R⁵ is hydrogen, and n=1, R³ is selected such that it is not hydrogen or p-chloro. It is also desirable in some embodiments that when E is NH, Ar is phenyl or naphthyl, B is NR⁴, R⁴ is C₂-C₁₂alkyl or C₅-C₂₀ aryl-C₁-C₁₂ alkyl, R⁵ is hydrogen, n = 1, and R³ is hydrogen, halo, or C₁-C₁₂ alkyl, at least one R² is not hydrogen. Preferably at least one R² and at least one R³ are not hydrogen.

Particularly preferred compounds include those of general Formula II: 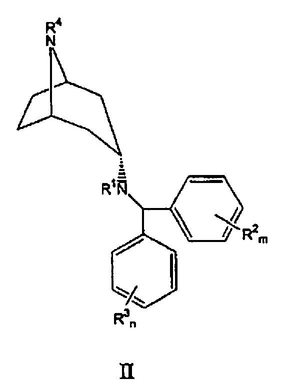 or a pharmaceutically acceptable salt or solvate thereof, wherein R¹ is hydrogen or methyl; m is 1 or 2; n is 1 or 2; R² and R³ are each independently fluoro, chloro, or hydrogen; and R⁴ is selected from the group consisting of hydrogen, methyl, n-butyl, allyl, phenylbutyl, 2-ethylamino, [2-(1*H*-indol-3-yl)-ethyl]-, and 3-[(N-phenyl)propionamido]. Exemplary compounds include those selected from the group consisting of:

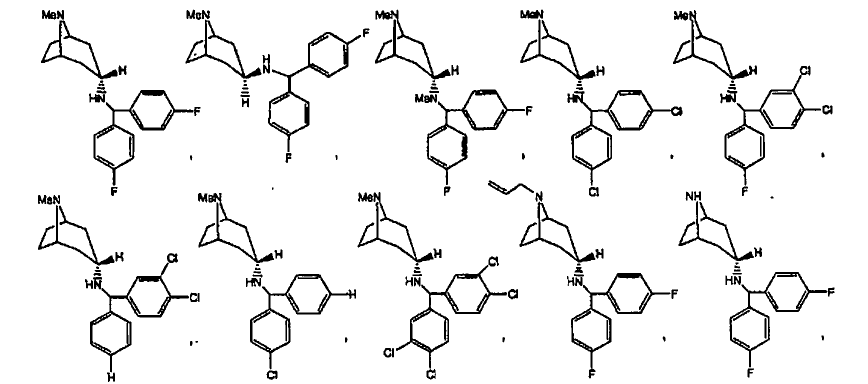

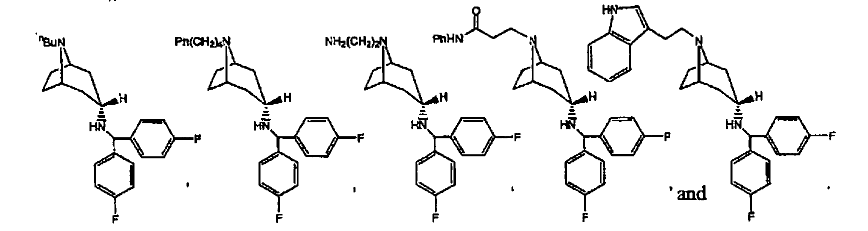

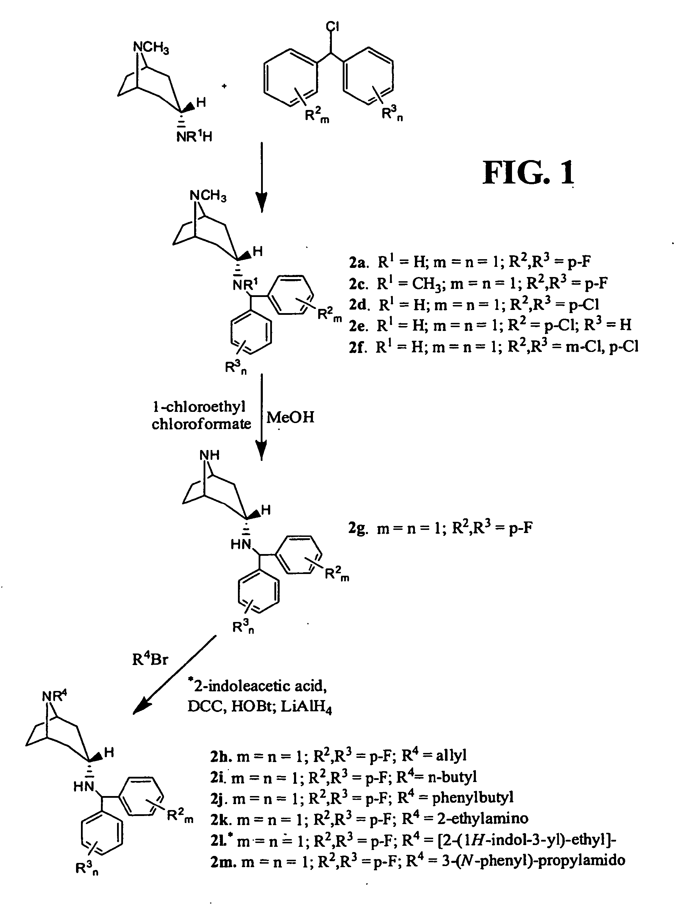

The compounds of Formulae I and II can be prepared by any suitable method, for example, by using the synthetic scheme set forth in FIG. 1. Briefly, the substituted benzhydrols are converted to the benzhydrylchlorides in refluxing thionyl chloride. The benzhydrylchlorides are then added, neat or in a minimal volume of anhydrous diethyl ether, to tropanamine to form the benztropinamine analogs of the present invention. Benztropinamines having α-configuration are obtained from α-tropanamine; benztropinamines having β-configuration are obtained from β-tropanamine.

The method for preparing a compound having the Formula I or II comprises (a) providing a benzhydrylhalide having the formula 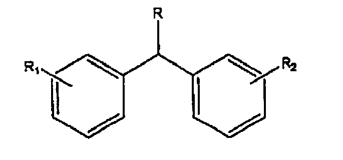 in which R is a halogen selected from the group consisting of Br, Cl, F and I; R₁ and R₂ are independently selected and are functional groups including, but not limited to, hydrogen, halo, C₁-C₁₂ alkyl, C₁-C₁₂ alkoxy, nitro, cyanato, isocyanato, thiocyanato, amino, halo C₁-C₁₂ alkyl, hydroxyl, trihalo C₁-C₁₂ alkyl, and any combination thereof; (b) adding the benzhydrylhalide to a tropanamine to form a reaction mixture; and (c) recovering the benzhopinamide analog from the reaction mixture. It will be readily apparent to those of skill in the art that the N-methyl group on the tropine can be substituted with other functional groups using standard chemical reactions known to and used by those of skill in the art. For example, using the reactions set forth in FIG. 1, the N-methyl group can be replaced with other functional groups including, but not limited to, hydrogen, C₂-C₁₂alkyl, C₂-C₁₂ alkenyl, C₂-C₁₂ alkynyl, C₅-C₂₀ aryl C₁-C₁₂ alkyl, C₅-C₂₀ heteroaryl C₁-C₁₂ alkyl, C₁-C₁₂ alkylamino, heterocyclyl C-₁C₁₂ alkyl, C₁-C₁₂ alkylsulfonyl, C₂-C₁₂ alkylcarbonyl, (*N*(C₅-C₂₀ aryl)amido)C₁-C₁₂ alkyl, and (*N*(C₁-C₁₀-alkyl)amido)C₁-C₁₂ alkyl. In particular, step (c) optionally further comprises the steps of demethylation and methanolysis of the tropine nitrogen followed by alkylation of the tropine nitrogen with an alkyl bromide of the formula R⁴Br. Alternatively, alkylation can occur upon reaction with the appropriate acid in the presence of DCC (dicyclohexylcarbodiimide) and HOBt (1-hydroxybenzotriazole hydrate). For example, the functional group R⁴ = [2-(1*H*-indol-3-yl)-ethyl]- can be obtained by reaction with 2-indoleacetic acid.

In order to understand the neurochemical and behavioral properties of the compounds of the present invention, exemplary benztropinamine analogs were evaluated for displacement of [³H]WIN 35,428 (2β-carbomethoxy-3β-(4-fluorophenyl)tropane) binding to the DAT in rat caudate-putamen. These benztropinamine analogs were also evaluated for displacement of radiolabeled ligand binding at the serotonin (SERT) and norepinephrine (NET) transporters as well as the muscarinic m₁ receptor (Ml), Table 1, infra. The NET over DAT selectivity refers to the Kᵢ ratio. When the Kᵢ ratio is greater than 1, the compound is DAT selective. The DAT selectivities of the analogs over these binding sites are depicted in Table 2.

**Table 1. Inhibition constants (Kᵢ) at the monoamine transporters and the muscarinic M1 receptor in rat brain membranes^{a}**

| | | | | | | | | |
|---|---|---|---|---|---|---|---|---|
| 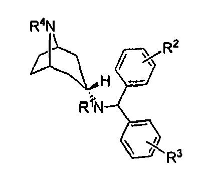 | | | | | | | | |

| Compound | R¹ | R² | R³ | R⁴ | DAT [³H]WIN 35,428 Kᵢ±SEM(nM) | SERT [³H]citalopram Kᵢ±SEM(nM) | NET [³H]nisoxetine Kᵢ ± SEM (nM) | M1 [³H]pirenzepine Kᵢ ± SEM (nM) |
|---|---|---|---|---|---|---|---|---|
| **2a** | H | 4-F | 4-F | CH₃ | 11.3 ± 1.61 | 8685 ± 436 | 1810 ± 269 | 7.81 ± 1.17 |
| **2b^{b}** | H | 4-F | 4-F | CH₃ | 661 ± 35 | 48500 ± 6370 | 15200 ± 1230 | 59.4 ± 7.3 |
| **2c** | CH₃ | 4-F | 4-F | CH₃ | 123 ± 15.5 | 17200 ± 2460 | 8410 ± 724 | 18.6 ± 2.48 |
| **2d** | H | 4-Cl | 4-Cl | CH₃ | 38.1 ± 5.35 | 4180 ± 623 | 7580 ± 325 | 50.0 ± 7.10 |
| **2e** | H | 4-Cl | H | CH₃ | 36.5 ± 0.66 | 13600 ± 1040 | 3170 ± 233 | 9.3 ± 0.27 |
| **2f** | H | 3,4-Cl | 3,4-Cl | CH₃ | 5.35 ± 0.25 | 3010 ± 332 | 259 ± 33.6 | 17.8 ± 2.27 |
| **2g** | H | 4-F | 4-F | H | 8.45 ± 0.23 | 4150 ± 368 | 997 ± 107 | 154 ± 19.6 |
| **2h** | H | 4-F | 4-F | allyl | 26.8 ± 3.43 | 3920 ± 581 | 5580 ± 821 | 130 ± 10.5 |
| **2i** | H | 4-F | 4-F | n-butyl | 21.5 ± 2.31 | 2640 ± 27.6 | 2920 ± 209 | 454 ± 36.7 |
| **2j** | H | 4-F | 4-F | phenylbutyl | 11.7 ± 0.39 | 502 ± 68.1 | 1630 ± 115 | 438 ± 56.2 |
| **2k** | H | 4-F | 4-F | 2-ethylamino | 12.5 ± 1.73 | 10900 ± 1090 | 3550 ± 222 | 2110 ± 119 |
| **2l** | H | 4-F | 4-F | [2-(1*H*-indol-3-yl)-ethyl]- | 64.5 ± 4.32 | 347 ± 413 | 8250 ± 939 | 413 ± 6.55 |
| **2m** | H | 4-F | 4-F | 3-[(N-phenyl)propionamido] | 4.61 ± 0.54 | 608 ± 41 | 2470 ± 83.3 | 2540 ± 124 |

| | | | | | | | | |
|---|---|---|---|---|---|---|---|---|
| ^{a}Each Kᵢ value represents data from at least three independent experiments, each performed in triplicate. Kᵢ values were analyzed by PRISM. A detailed description of the binding assay methods have been previously published (see Newman et al., J. Med. Chem. 44, 633 (2001); Houlihan et al., Medicinal Chemistry Research, 8, 77 (1998)). ^{b}The configuration at C-3 is β. | | | | | | | | |

**Table 2. Binding selectivities for the benztropinamine analogs**

| Compound | SERT/DAT | NET/DAT | M1/DAT |
|---|---|---|---|
| **2a** | 769 | 160 | 0.7 |
| **2b** | 73 | 23 | 0.1 |
| **2c** | 140 | 68 | 0.2 |
| **2d** | 110 | 199 | 1 |
| **2e** | 373 | 87 | 0.3 |
| **2f** | 563 | 48 | 3 |
| **2g** | 491 | 118 | 18 |
| **2h** | 146 | 208 | 5 |
| **2i** | 123 | 136 | 21 |
| **2j** | 43 | 139 | 37 |
| **2k** | 872 | 284 | 169 |
| **2l** | 5 | 128 | 6 |
| **2m** | 132 | 536 | 551 |

All of the compounds displaced [³H]WIN 35,428 binding at the dopamine transporter with a wide range of affinities (Kᵢ = 4.61 to 661 nM). The most potent compound in this exemplary series is compound **2m**, Kᵢ = 4.61 nM.

A comparison between compound **2a** and compound **2b** reveals that configuration at C-3 plays a pivotal role for the binding affinity at the DAT and to a somewhat lesser extent at the SERT, NET and the M1 receptor. As such, compared to **2a**, the binding affinity of **2b** at the DAT is reduced by 58-fold, while for the binding affinity at the SERT, the NET, and the M1 receptor a 6- to 8-fold lower binding affinity was found.

The introduction of a methyl group at the benzhydryl nitrogen, resulting in the tertiary amine **2c**, reduces binding affinity at the DAT by 11-fold compared to the secondary amine **2a.** The binding affinities at the SERT and the M1 receptor (about 2-fold lower) are far less affected by this modification than the binding affinity at the NET (about 5-fold lower).

The impact of the substitution patterns on the benzhydryl moiety on the binding affinity at the monoamine transporters and the M1 receptor was evaluated for analogs **2d, 2e,** and **2f.** Compared to the 4,4'-difluoro analog **2a,** 4,4'-dichloro (**2d**) as well as 4-chloro substitution (2e) reduce binding affinity at the DAT by around 3-fold. Only the derivative **2f,** with a 3,4-dichloro substitution on both phenyl rings, has a somewhat higher binding affinity at the DAT (2-fold) and a higher selectivity M1 over DAT than **2a.** However selectivity NET over DAT appears to be reduced in this case by 3-fold.

In compounds **2g, 2h, 2i, 2j, 2k,** and **2m,** alkylation at N-8 is generally well tolerated at the DAT and leads to a higher M1 over DAT selectivity profile than the parent compound **2a.** Furthermore these N-8 modified compounds show a retained if not higher NET over DAT selectivity. However, with the exception of **2k,** all of these compounds demonstrate a somewhat lower SERT over DAT selectivity than **2a.** Compound **2m** exhibits the highest DAT binding affinity (Kᵢ = 4.61 nM) and the highest NET over DAT and M1 over DAT selectivities in this series (535-fold and 551-fold, respectively). The N-8 ethylamino derivative **2k** shows a similar binding affinity at the DAT as the parent compound **2a.** The SERT over DAT and NET over DAT selectivity profile is retained, if not somewhat improved. Compared to **2a,** the binding affinity at the muscarinic M1 receptor is greatly reduced (170-fold). The indole derivative **21** differs from the rest of the N-8 modified derivatives (**2g, 2h, 2i, 2j, 2k** and **2m**) somewhat, for example, its binding affinity at the DAT is 6-fold lower than the parent compound **2a.** Interestingly, **21,** presumably due to the indole moiety as serotonin transporter pharmacophore, showed by far the lowest SERT over DAT selectivity in this series.

The benztropinamine analogs of the present invention find use as therapeutics for the treatment of a mental disorders, e.g., those selected from the group consisting of conduct disorders, alcohol addiction, tobacco addiction, nicotine addiction, drug addiction, sleep disorders, inhalation disorders, Parkinsonism including Parkinson's disease, female and male orgasmic disorders, female and male sexual arousal disorders, hypoactive sexual desire disorder, and anxiety and/or depression disorders. Preferably, the benztropinamine analogs are used to treat cocaine abuse, narcolepsy, or Attention Deficit Hyperactivity Disorder (ADHD). More preferably, the benztropinamine analogs are used to treat cocaine abuse.

These compounds inhibit dopamine uptake and provide elevated levels of extracellular dopamine that alleviate the symptoms of cocaine abstinence (see, Rothman et al., Life Sci. Pharmacol. Lett. 1990, 46, PL-17-PL-21) in a manner similar to the way in which the nicotine patch or nicotine chewing gum protects against withdrawal symptoms after cessation of tobacco use. Further, as a result of their lack of cocaine-like behavioral effects, these compounds are not subject to abuse themselves. Thus, the benztropinamine analogs of the present invention can serve to keep drug abusers from seeking cocaine, but they will not become substitute addictive drugs.

As used herein, "cocaine abuse" has its conventional meaning, i.e., misuse or addiction of cocaine. Typically, cocaine is taken by a person due to a craving for cocaine generated by its prior use. Cocaine is abused when it is used for gratification, producing effects not required or recommended for therapy. The resultant high use of cocaine produces many serious and adverse side effects. As such, it is highly desirable to reduce the number and/or intensity of episodes in which a person experiences a craving for the substance or, more preferably, to eliminate the craving episodes entirely. Craving is associated with decreased dopamine levels in the brain that result in feelings of dysphoria. If dopamine levels remain elevated, craving will be alleviated and the cocaine abuser will not seek cocaine. As such, compounds that increase dopamine levels for a prolonged period of time without causing euphoria and reinforcement that would lead to abuse would provide a therapeutic treatment for cocaine addiction.

The ability of these compounds to inhibit dopamine uptake and likewise cause increased and sustained dopamine levels in the brain is also useful for treating other addiction and mental disorders including alcohol addiction (see Eiler et al., Synapse, 489, 45 (2003); Tupala et al., Neuroimage, 19, 145 (2003)), nicotine addiction (see Bahk et al., Prog. Neuropsychopharmacol. Biol. Psychiatry, 26, 1095 (2002); Geracioti et al., Am. J. Psychiaty, 156, 130 (1999)); other types of drug addiction (see Campiani et al., J. Med. Chem., 46, 3822 (2003); Chartoff et al., J. Neurochem., 38, 107 (2003); O'Shea et al., Trends Pharmacol. Sci., 24, 272 (2003)); sexual dysfunction including orgasmic disorder, female/male sexual arousal disorders, and hypoactive sexual desire disorder (see Earler et al., Urology, 62, 727 (2003); Guiliano et al., Eur. Urol., 40, 601 (2001)); sleep disorders including narcolepsy and cataplexy (see Wisor et al., J. Neurosci., 21, 1787 (2001); Honda et al., Neuroreport, 10, 3713 (1999)); parkinsonism including Parkinson's disease (see Moore, Parkinsonism Relat. Disord., 9 Suppl. 2, S65 (2003); Stocchi et al., J. Neurol., 250, 822 (2003)); conduct disorders including ADHD (see Young et al., Am. J. Med. Genet., 114, 144 (2002); Seeman et al., Behav. Brain Res., 130, 79 (2002); Solanto, Behav. Brain Res., 130, 65 (2002); Swanson et al., Behav. Brain Res., 130, 73 (2002)); and depression, anxiety, and stress disorders (see Wall et al., Prog. Neuropsychopharmacol. Biol. Psychiatry, 27, 395 (2003); Laasko et al., Am. J. Psychiatry, 160, 904 (2003); Lawford et al., Eur. Neuropsychopharmacol., 13, 313 (2003); Buller et al., Psychoneuroendocrinology, 28, 715 (2003); Brunswick et al., Am. J. Psychiatry, 160, 1836 (2003); Kondo et al., Prog. Neuropsychopharmacol. Biol. Psychiatry, 27, 921 (2003)).

As such, in another aspect, the present invention provides a method of treating a patient for a mental disorder, the method comprising administering to the patient a therapeutically effective amount of a compound of Formula I or II.

"Treatment" or "treating," as used herein, refer to any administration of a compound of the present invention and include: (i) inhibiting the symptoms of the mental disorder, e.g., cocaine addiction; and/or (ii) lessening or inhibiting the long term effects of the mental disorder, e.g., cocaine addiction. In therapeutic applications, compositions are administered to a patient already suffering from the mental disorder, e.g., cocaine addiction, in an amount sufficient to cure or at least partially arrest or alleviate the symptoms of the mental disorder and/or its complications. An amount adequate to accomplish this is defined as a "therapeutically effective" amount or dose. Amounts effective for this use will depend on the severity and course of the mental disorder, previous therapy, the patient's health status and response to the drugs, and the judgment of the treating physician.

In conjunction with the foregoing method, the present invention provides pharmaceutical compositions comprising a compound of Formula I or II and a pharmaceutically acceptable carrier, diluent, or excipient. The phrase "pharmaceutically or therapeutically acceptable carrier," as used herein, refers to a carrier medium which does not interfere with the effectiveness of the biological activity of the active ingredients and which is not toxic to the host or patient. The pharmaceutical compositions of the present invention can be in a variety of forms. These include, for example, solid, semi-solid and liquid dosage forms, such as tablets, pills, powders, liquid solutions or suspensions, liposomes, injectable and infusible solutions. Inhalable preparations, such as aerosols, are also included. Preferred formulations are those directed to oral, intranasal and parenteral applications, but it will be appreciated that the preferred form will depend on the particular therapeutic application at hand. The methods for the formulation and preparation of therapeutic compositions comprising the compounds of the invention are well known in the art and are described in, for example, REMINGTON' S PHARMACEUTICAL SCIENCES (Mack Publishing Company, Philadelphia, Pa., 17th ed. (1985)), THE MERCK INDEX 11th Ed., (Merck & Co. 1989), and Langer, Science, 249: 1527-1533 (1990).

The pharmaceutical compositions containing the compounds of the present invention can be administered for therapeutic and/or prophylactic treatments. In therapeutic applications, compositions are administered to a patient already suffering from a mental disorder, e.g., cocaine addiction or Parkinson's disease, in an amount sufficient to cure or at least partially arrest the symptoms of the mental disorder and its complications.

In prophylactic applications, the pharmaceutical compositions are administered to a patient susceptible to or otherwise at risk for a particular disease in an amount sufficient to prevent or ameliorate the onset of symptoms. Such an amount is defined as a "prophylactically effective amount or dose." These can be administered orally or by inhalation. In this use, the precise amounts again depend on the patient's state of health, weight, and the like.

Once improvement of the patient's conditions has occurred, a maintenance dose is administered if necessary. Subsequently, the dosage or the frequency of administration, or both, can be reduced, as a function of the symptoms, to a level at which the improved condition is retained. When the symptoms have been alleviated to the desired level, treatment can cease. Patients can, however, require intermittent treatment on a long-term basis upon any recurrence of the mental disorder symptoms.

In general, a suitable effective dose of the compounds of the present invention will be in the range of 0.05 to 1000 milligram (mg) per recipient per day, preferably in the range of 0.1 to 100 mg per day. The desired dosage is preferably presented in one, two, three, four or more subdoses administered at appropriate intervals throughout the day. These subdoses can be administered as unit dosage forms, for example, containing 0.01 to 1000 mg, preferably 0.01 to 100 mg of active ingredient per unit dosage form. Again, the desired dosage will depend on, for example, the particular compound employed, the mental disorder to be treated, the manner of administration, the weight and general state of health of the patient, and the judgment of the prescribing physician.

While it is possible to administer the active ingredient of this invention alone, it is preferable to present it as part of a pharmaceutical formulation. The formulations of the present invention comprise at least one compound described herein in a therapeutically or pharmaceutically effective dose together with a pharmaceutically acceptable carrier. For parenteral administration, for example, the pharmaceutical compositions comprise a solution of a compound of Formula I or II, as described above, dissolved or suspended in an acceptable carrier, preferably an aqueous carrier. A variety of aqueous carriers can be used including, for example, water, buffered water, 0.4% saline, 0.3% glycine, hyaluronic acid and the like. These compositions may be sterilized by conventional, well known sterilization techniques or, they may be sterile filtered. The resulting aqueous solutions may be packaged for use as is or lyophilized, the lyophilized preparation being combined with a sterile solution prior to administration. The compositions may contain pharmaceutically acceptable auxiliary substances as required to approximate physiological conditions including pH adjusting and buffering agents, tonicity adjusting agents, wetting agents and the like, such as, for example, sodium acetate, sodium lactate, sodium chloride, potassium chloride, calcium chloride, sorbitan monolaurate, triethanolamine oleate, etc.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally about 10% to about 95% of the active ingredient and, more preferably, about 25% to about 75% of the active ingredient.

For aerosol administration, the compounds of Formula I or II are preferably supplied in a finely divided form along with a surfactant and propellant. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. A carrier can also be included as desired, as with, e.g., lecithin, for intranasal delivery.

In addition to the foregoing, the benztropinamine analogs of the present invention are useful as imaging probes for dopamine transporter/cocaine binding sites and as imaging probes for neurodegenerative disorders (e.g., Parkinson's disease). As such, in another aspect, the present invention provides a method of selectively imaging cocaine binding sites of the central nervous system of a human, the method comprising: (a) administering to the central nervous system of the patient a compound having the Formula I or II; and (b) detecting the binding of that compound to the central nervous system tissue.

Also disclosed is a method of detecting or monitoring parkinsonism in a patient, the method comprising: (a) administering to the human a detectably labeled compound having the Formula I or II; and (b) detecting the binding of that compound to the central nervous system tissue. Using this method, one can diagnose and/or monitor Parkinson's disease, a neurological disorder characterized by the progressive degeneration of dopamine nerve terminals.

The previous discussion pertaining to various embodiments including preferred embodiments of benztropinamine analogs as compounds per se is applicable to the benztropinamine analogs used in the method of imaging cocaine binding sites and in the methods of diagnosing/monitoring parkinsonism and, thus, they will not be repeated herein. In an embodiment, the benztropinamine analogs of the present invention are labeled with a radioactive or fluorescent label using standard labeling techniques known to and used by those of skill in the art. Suitable labels include, but are not limited to ¹¹C on the N-linked R⁴ substituent; ¹²³I, ⁷⁶Br or ¹⁸F, in an embodiment, on the phenylarylmethyl group attached to E; and ⁹⁹Tc on the phenylarylmethyl group attached to E.

In addition, in an embodiment, binding of the benztropinamine analogs to the CNS tissue is detected using positron emission tomography (PET) or single-photon emission computed tomography (SPECT). PET imaging may be carried using any appropriate apparatus, but is preferably carried out using coded single ring positron tomograph (Brownell et al., Intl. J. Imaging Syst. Tech., 1: 207-217, 1989). The analog ring design offers a number of advantages for positron tomography. PET imaging can be carried out on conscious human subjects. In addition, SPECT imaging may also be used on human subjects (See, e.g., Medicine, Scientific American, Inc., ed. Rubenstein and Federman, 1988; Jaszczak and Coleman, Invest. Radiol., 20: 897, 1985; and Coleman, et al., Invest. Radiol., 21: 1, 1986); preferably SPECT imaging employs gamma-emitting derivatives of the analogs described herein (e.g., benztropinamine analogs labeled with ¹²³I or ⁹⁹Tc).

As such, using the benztropinamine analogs of the present invention, one can (1) assay cocaine receptors in chronic cocaine users and in individuals exposed to cocaine prenatally, (2) assay the receptor occupancy of potential cocaine therapeutics, (3) assay cocaine receptors in individuals that abuse other drugs, (4) investigate the mechanism by which cocaine and related drugs alter behavior, (5) elucidate the receptor properties of the dopamine transporter receptor complex, (6) study the mechanism of dopamine transport, etc. Thus, the benztropinamine analogs of the present invention are useful, *inter alia,* in research, e.g., in *in vivo* and *in vitro* experiments, to study dopamine transport, the dopamine transport receptor and, in particular, cocaine binding sites.

The invention will be described in greater detail by way of specific examples.
The following examples are offered for illustrative purposes, and are intended neither to limit nor define the invention in any manner.

### EXAMPLES

**General Methodology.** All melting points were determined on a Thomas-Hoover melting point apparatus and are uncorrected. The ¹H and ¹³C NMR data were recorded on a Varian Mercury Plus 400 instrument. Samples were dissolved in an appropriate deuterated solvent. Proton chemical shifts are reported as parts per million (δ) relative to tetramethylsilane (Me₄Si; 0.00 ppm) which was used as an internal standard. Carbon chemical shift values (δ) are reported in parts per million (ppm) relative to deuterated chloroform (CDCl₃; 77.0 ppm). Mass spectra were recorded on a Hewlett Packard (Palo Alto, Calif.) 5971A mass selective ion detector in the electron-impact mode with sample introduction via a HP-5890 series II, gas chromatograph fitted with an HP-1 (crosslinked methyl silicone gum) 25 meters x 0.2 mm i.d., 50 micron film thickness. Ultrapure grade helium was used as the carrier gas at a flow rate of 1.2 ml/min. The injection port and transfer line temperatures were 250 °C and 280 °C, respectively. The initial oven temperature was 100 °C, held for 3.0 min, programmed to 295 °C at 15.0 °C/min, maintained at 295 °C for 10.0 min. Infrared spectra were recorded in KBr with a Perkin-Elmer 1600 Series FTIR. Microanalyses were performed by Atlantic Microlab, Inc. (Norcross, Ga.) and agree within 0.4% of calculated values. All chemicals and reagents were purchased from Aldrich Chemical Co. or Lancaster Synthesis, Inc.

### EXAMPLE 1

**Synthesis of Benztropinamines (2a-m)-General Method.** The appropriate benzhydrol was dissolved in 10 ml SOCl₂, at 0 °C, under an atmosphere of argon. The reaction mixture was warmed to reflux and allowed to stir at this temperature for 2-18 h. The reaction flask was cooled in an ice bath and the volatiles were removed *in vacuo.* Addition of dry toluene (2 x 5 ml) and removal *in vacuo* ensured the complete removal of SOCl₂. The resulting viscous oil was determined spectroscopically to be the desired benzhydryl chloride. The resulting benzhydrylchloride (1.1 eq.) was added to the appropriate 3-aminotropane and NaHCO₃ (2.5 eq) in acetonitrile and allowed to stir at reflux for 16 h. Upon completion of the reaction the volatiles were removed *in vacuo,* and the residue was purified by flash chromatography followed by cystallization of the appropriate salt.

**General procedure for N-alkylations**: A suspension of 1.0 eq. amine, sodium bicarbonate (2.5 eq.) and the appropriate 1.2 eq. alkyl bromide or chloride (in case of an alkyl chloride, generally a spatula tip of potassium iodide was added to the reaction mixture) in 10 ml acetonitrile was heated to 120 °C in a sealed tube for 16 h. Upon completion of the reaction, the volatiles were removed *in vacuo* and the residue was purified by flash chromatography followed by cystallization of the appropriate salt.

**Compound 2a**. Prepared from 3α-aminotropane (see Berdini et al., Tetrahedron, 58, 5669 (2002)) and 4,4'-difluorobenhydryl chloride according to the general procedure. Yield: 71%. M.p. (oxalate, ethanol/diethyl ether) 166 °C. *R*_{f} 0.26 (chloroform/methanol 10:1, 1% ammonium hydroxide). IR (film): v 3303. ¹H NMR (400 MHz, CDCl₃): δ 1.57 (d, J 13.2, 2H), 1.96-2.05 (m, 6H), 2.25 (s, 3H), 2.74 (t, J 6.6, 1H), 3.09 (s, 2H), 4.85 (s, 1H), 6.93-6.97 (m, 4H), 7.18-7.22 (m, 4H). ¹³C NMR (101 MHz, CDCl₃): δ 26.97, 37.05, 40.92, 46.92, 60.84, 63.29, 115.31 (J_{CF} 21), 128.91 (J_{CF} 8), 139.67 (J_{CF} 3), 161.44 (J_{CF} 243). Anal. (C₂₁H₂₄F₂N_{2·}2(COOH)_{2·}H₂O) C, H, N.

**Compound 2b.** Prepared from 3β-aminotropane (see Lewin et al., J. Med. Chem., 35, 135 (1992)) and 4,4'-difluorobenzhydryl chloride according to the general procedure. Yield: 44%. M.p. (oxalate, ethanol) 206-208 °C. IR (film): v 3284. ¹H NMR (400 MHz, CDCl₃): δ 1.30 (m, 2H), 1.44 (m, 2H), 1.79 (m, 2H), 1.90 (m, 2H), 2.24 (s, 3H), 2.61 (m, 1H), 3.12 ("t", "J" 3.2, 2H), 4.94 (s, 1H), 6.92-6.96 (m, 4H), 7.26-7.30 (m, 4H). ¹³C NMR (101 MHz, CDCl₃): δ 27.19, 39.77, 40.43, 46.52, 61.46, 62.80, 115.47 (J_{CF} 21), 128.72 (J_{CF} 8), 140.04 (J_{CF} 3), 161.64 (J_{CF} 243). Anal. (C₂₁H₂₄F₂N_{2·}2(COOH)₂) C, H, N.

**Compound 2c**. Prepared from 3α-methylaminotropane (see Archer et al., J. Am. Chem. Soc., 79, 4194 (1957)) and 4,4'-difluorobenzhydryl chloride according to the general procedure. Yield: 52%. M.p. (oxalate, acetone/diethyl ether) 227-228 °C. *R*_{f} 0.41 (chloroform/methanol 5:1, 1 % ammonium hydroxide). ¹H NMR (400 MHz, CDCl₃): δ 1.78-1.89 (m, 4H), 2.03-2.16 (m, 4H), 2.05 (s, 3H), 2.24 (s, 3H), 2.81 (m, 1H), 3.18 (t, J 4.4, 2H), 5.07 (s, 1H), 6.94-7.00 (m, 4H), 7.18-7.22 (m, 4H). ¹³C NMR (101 MHz, CDCl₃): δ 27.68, 30.26, 34.41, 38.39, 45.70, 5.58, 64.13, 115.31 (J_{CF} 21), 130.33 (J_{CF} 8), 137.23 (J_{CF} 3), 161.93 (J_{CF} 245). Anal. (C₂₂H₂₆F₂N₂·C₂H₂O_{4·}0.75H₂O) C, H, N.

**Compound 2d.** Prepared from 3α-aminotropane and 4,4'-dichlorobenzhydryl chloride according to the general procedure. Yield: 68%. M.p. (DL-tartrate, acetone/diethyl ether) 187-189 °C. *R*_{f} 0.21 (chloroform/methanol 10:1, 1% ammonium hydroxide). IR (film): v 3261. ¹H NMR (400 MHz, CDCl₃): δ 1.38 (s, br., 1H), 1.56 (d, J 14.0, 2H), 1.95-2.03 (6H), 2.73 (m, 1H), 3.08 (s, 2H), 3.24 (s, 3H), 4.84 (d, J 5.6, 1H), 7.19 (d, J 8.8, 4H), 7.26 (d, J 8.4, 4H). ¹³C NMR (101 MHz, CDCl₃): δ 26.63, 36.88, 40.76, 46.79, 60.62, 63.31, 128.03, 129.02, 132.92, 142.52. Anal. (C₂₁H₂₄Cl₂N₂·2(CHOHCOOH)_{2·}0.5H₂O)C,H,N.

**Compound 2e**. Prepared from 3α-aminotropane and 4-monochloro-benzhydryl chloride according to the general procedure. Yield: 47%. *R*_{f} 0.36 (chloroform/methanol 10:1, 1% ammonium hydroxide). ¹HNMR (400 MHz, CDCl₃): δ 1.41 (s, 1H), 1.58 (m, 2H), 1.99-2.08 (m, 6H), 2.27 (s, 3H), 2.79 (t, J 6.5, 1H), 3.11 (s, 2H), 4.87 (s, 1H), 7.21-7.33 (m, 9H). ¹³C NMR (101 MHz, CDCl₃): δ 26.16, 26.58, 26.63, 36.64, 36.95, 40.71, 46.83, 60.84, 64.03, 127.54, 127.94, 128.96, 128.99, 129.12, 129.42, 132.95, 143.31, 144.28. Anal. (C₂₁H₂₅ClN₂·(CHOHCOOH)₂ C, H, N.

**Compound 2f.** Prepared from 3α-aminotropane and 3,4,3',4'-tetrachlorobenzhydryl chloride according to the general procedure. Yield: 34%. *R*_{f} 0.36 (chloroform/methanol 10:1, 1 % ammonium hydroxide). ¹H NMR (400 MHz, CDCl₃): δ 1.44 (d, J 3.5, 1H), 1.62 (m, 1H), 1.98-2.20 (m, 6H), 2.35 (s, 3H), 2.82 (s, 1H), 3.23 (s, 2H), 4.83 (d, d 7.4, 1H), 7.13 (dd, J 8.2, 1.6, 1H), 7.21-7.41 (m, 5H). ¹³C NMR (101 MHz, CDCl₃): δ 26.25, 26.34, 35.94, 36.51, 40.40, 46.53, 61.14, 63.70, 127.36, 127.74, 127.80, 129.11, 129.80, 130.72, 131.11, 132.80, 143.29, 144.93. C₂₁H₂₄Cl₂N₂·2(COOH)₂·0.5H₂O C, H, N.

**Compound 2g.** To a suspension of 2a (0.38 g, 1.2 mmol) and sodium bicarbonate (0.5 g, 5.9 mmol) in 10 ml anhydrous 1,2 dichloroethane was added 1-chloroethyl chloro formate (0.3 ml, 2.7 mmol) at 0 °C and the mixture was refluxed under an atmosphere of argon for 4h. After that time the reaction mixture was filtered and all volatiles were removed *in vacuo.* The residue was taken up in methanol (15 ml) and heated to a gentle reflux overnight. The solvent was removed in vacuo and the residue was basified to yield 0.36 g (92%) in for further conversions sufficient purity. An analytical pure sample, obtained by crystallization from isopropanol, was converted into the oxalic acid salt for pharmacological evaluation. M.p. (oxalate, ethanol) 138-140 °C. *R*_{f} 0.47 (chloroform/methanol/triethylamine 5:1:1). ¹H NMR (400 MHz, CDCl₃): δ 1.40 (s, br., 1H), 1.63 (d, J 13.6, 2H), 1.87 (m, 2H), 1.99 (m, 2H), 2.15 (m, 2H), 2.83 ("t", J 5.6, 1H), 3.39 ("s", 1H), 3.55 ("s", 2H), 4.87 (d, J 4.4, 1H), 6.96-7.00 (m, 4H), 7.20-7.24 (m, 4H). ¹³C NMR (101 MHz, CDCl₃): δ 29.76, 37.28, 47.22, 54.13, 63.16, 115.49 (J_{CF} 21), 129.13 (J_{CF} 8), 139.80 (J_{CF} 3), 161.89 (J_{CF} 245). Anal. (C₂₀H₂₂F₂N_{2·}2(COOH)_{2·}0.5 H₂O.

**Compound 2h.** Prepared from **2g** and allyl bromide according to the general procedure. Yield: 65 %. M.p. (hydrochloride, isopropanol/diethyl ether) 184 °C. *R*_{f} 0.27 (chloroform/methanol 10:1, 1% triethylamine). ¹H NMR (400 MHz, CDCl₃): δ 1.42 (s, br., 1H), 1.67 (d, J 14.0, 2H), 1.99-2.04 (m, 2H),2.16-2.29 (m, 4H), 2.87 (t, J 6.4, 1H), 3.16 (d, J 6.4, 2H), 3.96 ("s", 1H), 4.85 ("s", 1H), 5.23 (m, 2H), 6.04 (m, 1H), 6.95-7.01 (m, 4H), 7.18-7.24 (m, 4H). ¹³C NMR (101 MHz, CDCl₃): δ 26.40, 31.13, 41.32, 51.06, 57.16, 63.15, 115.55 (J_{CF} 21), 119.61, 129.13 (J_{CF} 8), 133.62, 139.62 (J_{CF} 3.0), 161.90 (J_{CF} 244). Anal. (C₂₃H₂₆F₂N_{2·}1.5(CHOHCOOH)_{2·}H₂O) C, H, N.

**Compound 2i.** Prepared from **2g** and butyl bromide according to the general procedure. Yield: 58%. M.p. (hydrochloride, isopropanol/diethyl ether) 142-143 °C. *R*_{f} 0.24 (ethylacetate). ¹H NMR (400 MHz, CDCl₃): δ 0.91 (m, 3H), 1.26-1.54 (m, 6H), 1.96-2.00 (m, 4H), 2.29-2.34 (m, 2H), 2.75 (s, 1H), 3.17 ("s", 2H), 4.86 (d, J 6.4, 1H), 6.95-7.01 (m, 4H), 7.21-7.26 (m, 4H). ¹³C NMR (101 MHz, CDCl₃): δ 14.75, 27.28, 31.73, 36.32, 47.37, 52.20, 58.55, 63.30, 115.59 (J_{CF} 21), 129.31 (J_{CF} 8), 140.23 (J_{CF} 2), 162.02 (J_{CF} 245). Anal. (C₂₄H₃₀F₂N_{2·}1.5(CHOHCOOH)_{2·}0.5H₂O) C, H, N.

**Compound 2j.** Prepared from **2g** and (4-iodo-butyl)-benzene according to the general procedure. Yield: 61%. M.p. (oxalate, ethanol) 194-196 °C. *R*_{f} 0.68 (chloroform/methanol 10:1, 1% ammonium hydroxide). ¹HNMR (400 MHz, CDCl₃): δ 1.37 (s, 1H), 1.47-1.65 (m, 6H), 1.92-2.03 (m, 6H), 2.33 (t, J 7.6, 2H), 2.61 (t, J 7.6, 2H), 2.74 ("s", 1H), 3.15 (s, 2H), 4.85 (d, J 5.8, 1H), 6.95-6.99 (m, 4H), 7.13-7.17 (m, 3H), 7.20-7.27 (m, 6H). ¹³C NMR (101 MHz, CDCl₃): δ 27.13, 29.08, 30.00, 36.29, 36.37, 47.25, 52.29, 58.59, 63.26, 115.69 (J_{CF} 21), 126.09, 128.71, 128.85, 129.44 (J_{CF} 8), 140.36 (J_{CF} 3), 143.06, 162.23 (J_{CF} 245). Anal. (C₃₀H₃₄F₂N₂·(COOH)₂·H₂O) C, H, N.

**Compound 2k.** Prepared from **2g** and 2-(2-bromo-ethyl)-isoindole-1,3-dione according to the general alkylation procedure, followed by treatment of the intermediate with hydrazine. Yield: 51%. M.p. (oxalate, ethanol) 158-160 °C. *R*_{f} 0.16 (chlorofonn/methanol 10:1, 1% ammonium hydroxide). ¹H NMR (400 MHz, CDCl₃): δ 1.47 (s, 3H), 1.54 (d, J 13.8, 2H), 1.90-2.03 (m, 6H), 2.37 (t, 2H), 2.69-2.74 (m, 3H), 3.13 (s, 2H), 4.86 (s, 1H), 6.95-7.04 (m, 4H), 7.21-7.28 (m, 4H). ¹³C NMR (101 MHz, CDCl₃): δ 27.13, 36.89, 41.31, 50.19, 55.79, 59.15, 63.20, 115.66 (J_{CF} 21), 129.42 (J_{CF} 8), 140.32 (J_{CF} 3), 162.20 (J_{CF} 246). Anal. (C₂₂H₂₇F₂N₃ (2.5 COOH)₂·0.5H₂O) C, H, N.

**Compound 21**. Prepared in adaptation of a procedure described in Agoston et al., J. Med. Chem., 40, 4329 (1997). Yield: 51%. M.p. (oxalate, isopropanol) 178-180 °C. *R*_{f} 0.52 (chloroform/methanol 10:1, 1% ammonium hydroxide). ¹HNMR (400 MHz, CDCl₃): δ 1.39 (s, 1H), 1.57 (d, J 13.7, 2H), 1.98-2.10 (m, 6H), 2.71 (m, 2H), 2.80 (t, J 6.0, 1H), 2.95 ("t", J 8.2, 2H), 3.34 (s, 2H), 4.87 (s, 1H), 6.95-7.01 (m, 5H), 7.09 (m, 1H), 7.16 (td, J 7.0, 1.2, 1H), 7.23 (m, 4H), 7.32 (d, J 8.2, 1H), 7.58 (d, J 7.8, 1H), 8.18 (s, 1H). ¹³C NMR (101 MHz, CDCl₃): δ 25.05, 26.99, 35.87, 47.02, 53.06, 58.67, 63.09, 111.39, 114.71, 115.43, 115.63, 119.06, 119.44, 121.83, 122.16, 127.74, 129.21, 129.29, 136.46, 140.07, 140.10, 160.84, 163.27. Anal. C₃₀H₃₁F₂N₃·(2COOH)₂ C, H, N.

**Compound 2m.** Prepared from **2g** and 3-bromo-N-phenyl-propionamide (see Glennon et al., J. Med. Chem., 24, 678 (1981)) to the general procedure. Yield: 54%. *R*_{f} 0.42 (ethylacetate/triethylamine 10:1). IR (film): v 3313, 1674. ¹H NMR (400 MHz, CDCl₃): δ 1.44 (s, 1H), 1.72 (d, J 14.0, 2H), 2.00-2.08 (m, 4H), 2.18 (m, 2H), 2.45 (t, J 5.6, 2H), 2.69 (t, J 5.6, 2H), 2.90 (t, J 6.0, 1H), 3.33 ("s", 2H), 4.89 (s, 1H), 7.19-7.25 (m, 6H), 7.47 (d, J 7.6, 2H), 7.98-7.25 (m, 5H), 11.53 (s, 1H). ¹³C NMR (101 MHz, CDCl₃): 26.99, 34.52, 37.27, 47.22, 48.78, 58.55, 63.27, 115.74 (J_{CF} 21), 119.78, 123.82, 129.30, 129.31(J_{CF} 8), 139.14, 139.85 (J_{CF} 4), 162.1 (J_{CF} 245), 171.43. Anal. (C₂₉H₃₁F₂N₃O·2(CHOHCOOH)₂ C, H, N.

### EXAMPLE 2

**Dopamine Transporter Binding Assay.** Male Sprague-Dawley rats (200-250 g, Taconic, Germantown, NY) were decapitated and their brains removed to an ice-cooled dish for dissection of the caudate putamen. The tissue was homogenized in 30 volumes ice-cold modified sucrose using a Brinkman polytron and centrifuged at 20,000 x g for 10 min at 4° C. The resulting pellet was then washed two more times by re-suspension in ice-cold buffer and centrifugation at 20,000 x g for 10 min at 4 °C. Fresh homogenates were used in all experiments.

Binding assays were conducted in modified sucrose buffer on ice. The total volume in each tube was 0.5 ml and the final concentration of membrane after all additions was 0.5% (w/v) corresponding to 200-300 mg of protein/sample. Triplicate samples of membrane suspension were preincubated for 5 min in the presence or absence of the compound being tested. [³H]WIN 35,428 (2-β-carbomethoxy-3-β-(4-fluorophenyl)tropane 1,5-naphthalene disulfonate; specific activity 82.4 Ci/mmol, from New England Nuclear, Boston, MA, final concentration 1.5 nM) was added and the incubation was continued for 1 h on ice. The incubation was terminated by the addition of 3 ml of ice-cold buffer and rapid filtration through Whatman GF/B glass fiber filter paper (presoaked in 0.1 % BSA in water to reduce non-specific binding) using a Brandee Cell Harvester (Gaithersburg, MD). The filters were washed with three additional 3 ml washes and transferred to scintillation vials. Absolute ethanol (0.5 ml) and Beckman Ready Value Scintillation Cocktail (2.75 ml) were added to the vials which were counted the next day at an efficiency of about 36%. Under these assay conditions, an average experiment yielded approximately 6,000 dpm total binding per sample, and approximately 250 dpm non-specific binding, defined as binding in the presence of 100 µM cocaine. Each compound was tested with concentrations ranging from 0.01 nM to 100 µM for competition against binding of [³H]WIN 35,428, in three independent experiments, each performed in triplicate.

Saturation and displacement data were analyzed by the use of the nonlinear least squares curve-fitting computer program PRISM. Data from replicate experiments were modeled together to produce a set of parameter estimates and the associated standard errors of these estimates. In each case, the model reported fit significantly better than all others according to the F test at p<0.05. The Kᵢ values reported are the dissociation constants derived for the unlabeled ligands.

**[³H]Nisoxetine Binding Assay**. Membranes from frozen frontal cortex dissected from male Sprague-Dawley rats (Taconic Labs, Germantown, NY) were homogenized in 20 volumes (w/v) of 50 mM Tris containing 120 mM NaCl and 5 mM KCl (pH 7.4 at 25 °C), using a Brinkman Polytron (at setting 6 for 20 sec). The tissue was centrifuged at 50,000 x g for 10 min at 4 °C. The resulting pellet was resuspended in buffer and recentrifuged. The final pellet was resuspended in cold buffer to a concentration of 80 mg/ml (original wet weight). Ligand binding experiments were conducted in assay tubes containing 0.5 ml buffer, 0.5 nM [³H]nisoxetine (New England Nuclear, Boston, MA), and 8 mg frontal cortex tissue. The reaction was started with the addition of the tissue and the tubes were incubated for 60 min at 0-4 °C. The incubation was terminated by rapid filtration through Whatman GF/B filters, presoaked in 0.05% polyethylenimine, using a Brandel Cell Harvester (Brandel Instruments Gaithersburg, MD). The filters were washed twice with 5 ml cold buffer, transferred to scintillation vials to which Beckman Ready Safe was added. Nonspecific binding was determined using 1 µM desipramine. Data were analyzed using GraphPad Prism software (San Diego, CA).

**[³H]Citalopram Binding Assay.** Membranes from frozen rat midbrain were homogenized in 20 volumes (w/v) of 50 mM Tris containing 120 mM NaCl and 5 mM KCl (pH 7.4 at 25 °C), using a Brinkman Polytron (at setting 6 for 20 sec). The tissue was centrifuged at 20,000 x g for 10 min at 4 °C. The resulting pellet was resuspended in buffer and recentrifuged. The final pellet was resuspended in cold buffer to a concentration of 15 mg/ml (original wet weight). Ligand binding experiments were conducted in assay tubes containing 0.5 ml of buffer, 1.4 nM [³H]citalopram (New England Nuclear, Boston MA), and 1.5 mg midbrain tissue. The reaction was started with the addition of the tissue and the tubes were incubated for 60 min at 25 °C (room temperature). The incubation was terminated by rapid filtration through Whatman GF/B filters (presoaked in 0.3% polyethylenimine in water) using a Brandel Cell Harvester (Brandel Instruments Gaithersburg, MD). The filters were washed twice with 5 ml cold buffer, transferred to scintillation vials to which Beckman Ready Safe was added. Nonspecific binding was determined using 10 µM fluoxetine (RBI, Natick, MA). Data were analyzed using GraphPad Prism software (San Diego, CA).

**[³H]Pirenzepine Binding Assay.** Membranes from frozen rat brains excluding cerebellum were thawed in ice-cold buffer (10 mM Tris-HCl, 320 mM sucrose, pH 7.4) and homogenized with a Brinkman polytron in a volume of 10 ml/gm of tissue. The homogenate was centrifuged at 1,000 x g for 10 min at 4 °C. The resulting supernatant was then centrifuged at 10,000 x g for 20 min at 4 °C. The resulting pellet was resuspended in a volume of 200 mg/ml in 10 mM Tris buffer (pH 7.4). Ligand binding assays were conducted in tubes containing 0.5 ml of buffer (10 mM Tris-HCl, 5 mM MgCl₂), 3 nM [³H]pirenzepine (New England Nuclear, Boston, MA), and 20 mg of brain tissue. The reaction was started with the addition of the tissue and the tubes were incubated for 60 min in a 37 °C water bath. The incubation was terminated by the addition of 5 ml of ice-cold buffer (10 mM Tris-HCl, pH 7.4) and rapid filtration through Whatman GF/B glass fiber filter paper (presoaked in 0.5% polyethylenimine) using a Brandel Cell Harvester (Brandel Instruments, Gaithersburg, MD). The filters were washed twice with 5 ml cold buffer, and transferred to scintillation vials to which absolute ethanol and Beckman Ready Safe was added. Quinuclidinyl benzilate (QNB), 100 µM final concentration, was used to determine non-specific binding. Data were analyzed by using GraphPad Prism software (San Diego, CA).

### EXAMPLE 3

This example demonstrates that compound **2m** lacks cocaine-like locomotor stimulant effects.

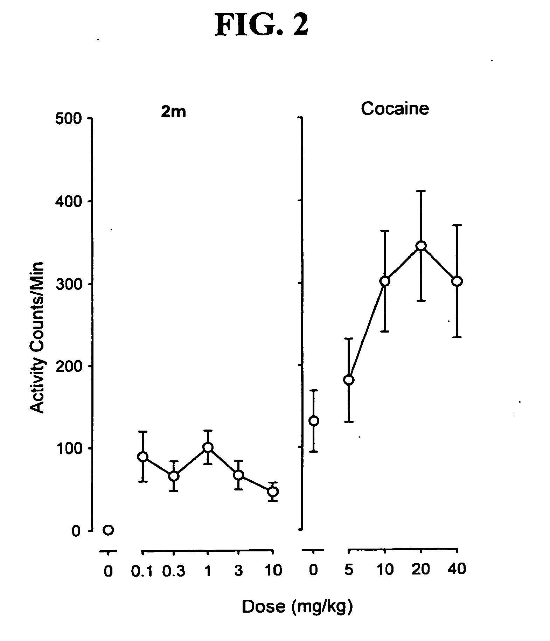

Mice were injected with various doses of either **2m** or cocaine and the number of activity counts per minute was measured (see FIG. 2). Cocaine, as has been shown in past studies produced dose-related increases in locomotor activity, approximating 400 counts per min at the most effective dose. In contrast, **2m** produced much more modest increases in activity of around 100 counts per min. In addition, the stimulant effects of **2m** were not related in an orderly way to dose, suggesting a non-specific pharmacological effect.

### EXAMPLE 4

This example demonstrates that compound **2m** lacks cocaine-like subjective effects.

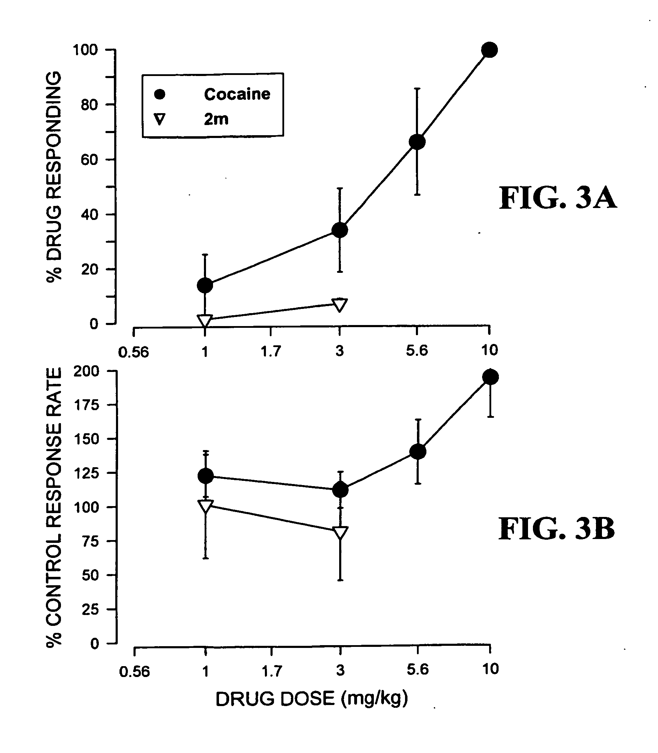

Rats were trained to emit one response after an injection of cocaine and a different response after injection of vehicle. Once the subjects were well trained, they act as detectors of cocaine-like subjective effects. Varying doses of cocaine and **2m** were injected into the rats, and the response rate was measured (see FIGS. 3A and 3B). Compound **2m** over the dose range tested did not substitute for cocaine.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

## Claims

1. A compound of the Formula I: 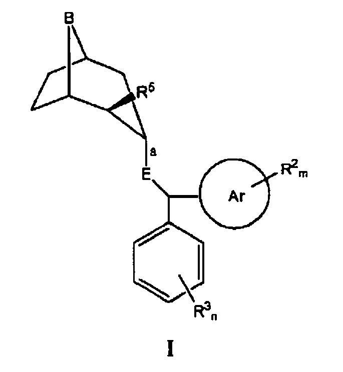 in which:
E is NR¹, S, or CH₂;
B is NR⁴, O, or CH₂;
R¹ is selected from the group consisting of hydrogen, C₁-C₁₂ alkyl, C₁-C₁₂ alkylamido C₁-C₁₂ alkyl, C₁-C₁₂ alkylamido C₅-C₂₀ aryl, C₂-C₁₂ alkylcarbonyloxy, C₂-C₁₂ alkoxyalkyl, C₁-C₁₂ hydroxyalkyl, C₃-C₁₂ alkylcarbonyloxyalkyl, C₅-C₂₀ aryl C₁-C₁₂ alkyl, C₅-C₂₀ aryloxy C₁-C₁₂ alkyl, cinnamyl, and C₂-C₁₂ alkylcarbonyl;
m = 1 to 5; n = 1 to 3;
R² and R³ are each independently selected from the group consisting of hydrogen, halo, C₁-C₁₂ alkyl, C₁-C₁₂ alkoxy, nitro, cyanato, isocyanato, thiocyanato, amino, halo C₁-C₁₂ alkyl, hydroxyl, trihalo C₁-C₁₂ alkyl, and any combination thereof;
R⁴ is selected from the group consisting ofhydrogen, C₂-C₁₂ alkyl, C₂-C₁₂ alkenyl, C₂-C₁₂ alkynyl, C₅-C₂₀ aryl C₁-C₁₂ alkyl, C₅-C₂₀ heteroaryl C₁-C₁₂ alkyl, C₁-C₁₂ alkylamino, heterocyclyl C₁-C₁₂ alkyl, C₁-C₁₂ alkylsulfonyl, C₂-C₁₂ alkylcarbonyl, (N(C₅-C₂₀ aryl)amido)C₁-C₁₂ alkyl, (N(C₁-C₁₂-alkyl)amido)C₁-C₁₂ alkyl, (N(C₅-C₂₀ aryl)amido)C₂-C₁₂ alkylcarbonyl (N(C₁-C₁₂-alkyl)amido)C₂-C₁₂ alkylcarbonyl and C₁-C₁₂ alkylamido C₅-C₂₀ aryl;
R⁵ is selected from the group consisting of hydrogen, hydroxyl, carboxyl, C₁-C₁₂ alkyl, C₁-C₁₂ alkoxy, C₂-C₁₂ alkylcarbonyl, C₂-C₁₂ carboxyalkyl, C₂-C₁₂ alkyloxycarbonyl, C₅-C₂₀ aryl, C₅-C₂₀ aryloxycarbonyl, C₅-C₂₀ aryl C₂-C₁₂ alkyloxycarbonyl, C₁-C₁₂ alkyl sulfonyl, C₁-C₁₂ hydroxyalkyl, formyl, C₂-C₁₂ formylalkyl, C₂-C₁₂ alkenyl, and C₂-C₁₂ alkynyl;
Ar is a C₅-C₂₀ monocyclic aryl group or a C₁₀-C₂₀ bicyclic aryl group or a heteroaryl group having 2 to 6 carbon atoms and one or more heteroatoms selected from the group consisting of N, O, S, and any combination thereof; and
bond "a" can be of α, β, or α/β configuration;
wherein any of R¹, R², R³, R⁴, and R⁵ other than hydrogen, halo, hydroxyl, nitro, cyanato, isocyanato, and thiocyanato may be further substituted with one or more substitutents selected from the group consisting of halo, hydroxyl, cyanato, isocyanato, thiocyanato, amino, C₁-C₁₂ alkyl, amido, nitro, and any combination thereof; or
a pharmaceutically acceptable salt or solvate thereof;
with the proviso that:
if bond "a" is of β configuration, Ar is phenyl, R² is hydrogen, B is NCH₂CH₃, E is NH, R⁵ is hydrogen, and n = 1, R³ is not hydrogen or p-chloro.

2. The compound, salt or solvate of claim 1, wherein E is NR¹.

3. The compound, salt or solvate of claim 2, wherein R¹ is hydrogen or C₁-C₁₂ alkyl, preferably C₁-C₆ alkyl.

4. The compound, salt or solvate of claim 1 or 2, wherein R³ is fluoro or chloro.

5. The compound, salt or solvate of any one of claims 1-4, wherein m = n = 1.

6. The compound, salt or solvate of any one of claims 1-5, wherein bond "a" is of α configuration.

7. The compound, salt or solvate of any one of claims 1-6, wherein Ar is selected from the group consisting of phenyl, naphthyl, biphenyl, pyridyl, bipyridyl, pyrimidyl, pyrrolyl, furanyl, thiophenyl, triazolyl, triazolopyrimidyl, thiadiazolyl, phosphole, diazaphosphole, quinoxalyl, benzofuranyl, benzopyrrolyl, morpholinyl, benzopyranyl, oxolyl, thiazolyl, purinyl, imidazolyl, indolyl, phosphindolyl, pyrazolyl, and isoindolyl, preferably phenyl.

8. The compound, salt or solvate of any one of claims 1-7, wherein R⁵ is H or C₂-C₁₂ alkylcarbonyloxy, preferably C₂-C₆ alkylcarbonyloxy.

9. The compound, salt or solvate of any one of claims 1-8, wherein B is NR⁴.

10. The compound, salt or solvate of claim 9, wherein R⁴ is selected from the group consisting of hydrogen, C₂-C₁₂ alkyl, C₂-C₁₂ alkenyl, C₅-C₂₀ aryl-C₁-C₁₂ alkyl, C₁-C₁₂ alkylamino, heterocyclyl-C₁-C₁₂ alkyl, and (N(C₅-C₂₀-aryl)amido)C₁-C₁₂ alkyl.

11. The compound, salt or solvate of claim 2, wherein:
R¹ is hydrogen or C₁-C₁₂ alkyl;
m=n= 1;
R² and R³ are halo, preferably chloro or fluoro, or hydrogen;
B is NR⁴;
R⁴ is selected from the group consisting of hydrogen, C₂-C₁₂ alkyl, C₂-C₁₂ alkenyl, C₅-C₂₀ aryl-C₁-C₁₂ alkyl, C₁-C₁₂ alkylamino, heterocyclyl-C₁-C₁₂ alkyl, and (N(C₅-C₂₀-aryl)amido)C₁-C₁₂ alkyl;
R⁵ is hydrogen;
Ar is phenyl; and
bond "a" is of α configuration.

12. The compound, salt or solvate of claim 2, wherein
R¹ is hydrogen or C₁-C₁₂ alkyl;
m = n = 2;
k² and R³ are halo, preferably chloro or fluoro, or hydrogen;
B is NR⁴;
R⁴ is selected from the group consisting of hydrogen, C₂-C₁₂ alkyl, C₂-C₁₂ alkenyl, C₅-C₂₀ aryl-C₁-C₁₂ alkyl, C₁-C₁₂ alkylamino, heterocyclyl-C₁-C₁₂ alkyl, and (N(C₅-C₂₀-aryl)amido)C₁-C₁₂ alkyl;
R⁵ is hydrogen;
Ar is phenyl; and
bond "a" is of α configuration.

13. The compound of claim 1, wherein the compound is of the Formula II: 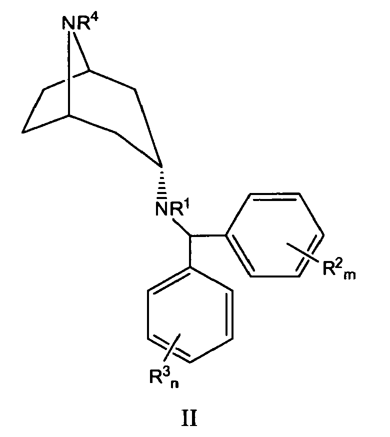 in which:
R¹ is hydrogen or methyl;
m = 1 or 2 and n = 1 or 2;
R² and R³ are each independently selected from hydrogen, fluoro, and chloro; and
R⁴ is selected from the group consisting of hydrogen, n-butyl, allyl, phenylbutyl, 2-ethylamino, [2-(1*H*-indol-3-yl)-ethyl]-, and 3-[(N-phenyl)propionamido];
or a pharmaceutically acceptable salt or solvate thereof.

14. The compound, salt or solvate of claim 13, wherein the compound is selected from the group consisting of:

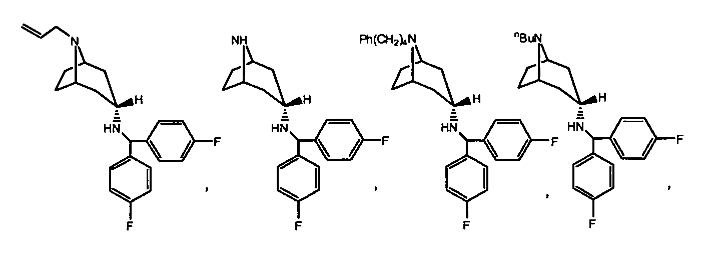

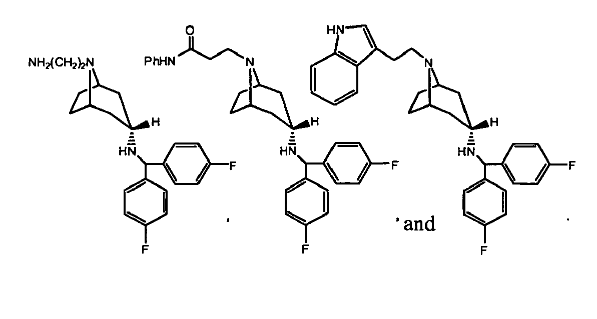

15. The compound, salt or solvate of claim 1, wherein R⁴ is C₂-C₁₂ alkyl.

16. A compound selected from the group consisting of: 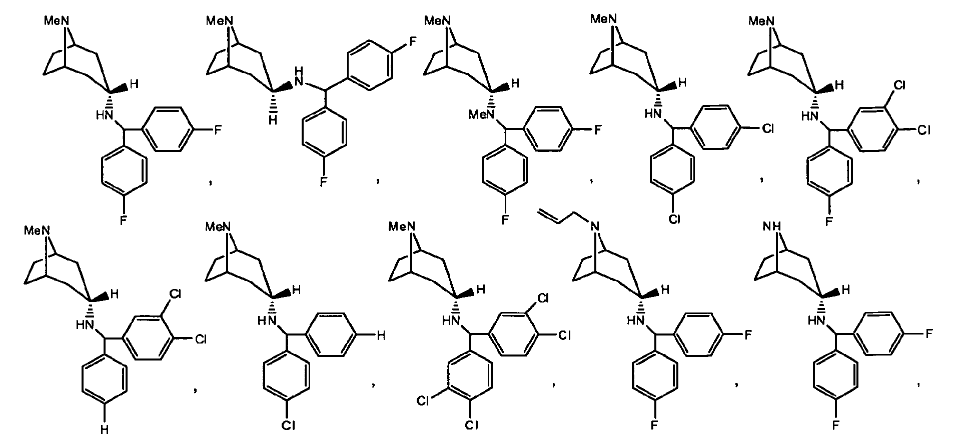 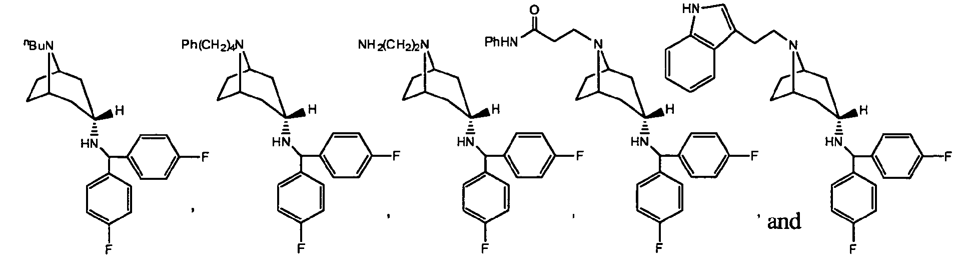 or a pharmaceutically acceptable salt or solvate thereof.

17. A compound of the Formula I: 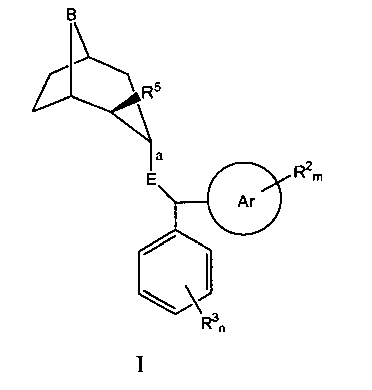 in which:
E is NR¹, S, or CH₂;
B is NR⁴, O, or CH₂;
R¹ is selected from the group consisting of hydrogen, C₁-C₁₂ alkyl, C₁-C₁₂ alkylamido C₁-C₁₂ alkyl, C₁-C₁₂ alkylamido C₅-C₂₀ aryl, C₂-C₁₂ alkylcarbonyloxy, C₂-C₁₂alkoxyalkyl, C₁-C₁₂ hydroxyalkyl, C₃-C₁₂ alkylcarbonyloxyalkyl, C₅-C₂₀ aryl C₁-C₁₂ alkyl, C₅-C₂₀ aryloxy C₁-C₁₂ alkyl, cinnamyl, and C₂-C₁₂ alkylcarbonyl;
m = 1 to 5; n = 1 to 3;
R² and R³ are each independently selected from the group consisting of hydrogen, halo, C₁-C₁₂ alkyl, C₁-C₁₂ alkoxy, nitro, cyanato, isocyanato, thiocyanato, amino, halo C₁-C₁₂ alkyl, hydroxyl, trihalo C₁-C₁₂ alkyl, and any combination thereof;
R⁴ is selected from the group consisting of hydrogen, C₁-C₁₂ alkyl, C₂-C₁₂ alkenyl, C₂-C₁₂ alkynyl, C₅-C₂₀ aryl C₁-C₁₂ alkyl, C₅-C₂₀ heteroaryl C₁-C₁₂ alkyl, C₁-C₁₂ alkylamino, heterocyclyl C₁-C₁₂ alkyl, C₁-C₁₂ alkylsulfonyl, C₂-C₁₂ alkylcarbonyl, (N(C₅-C₂₀ aryl)amido)C₁-C₁₂ alkyl, (N(C₁-C₁₂-alkyl)amido)C₁-C₁₂ alkyl (N(C₅-C₂₀ aryl)amido)C₂-C₁₂ alkylcarbonyl, (N(C₁-C₁₂-alkyl)amido)C₂-C₁₂ alkylcarbonyl and C₁-C₁₂ alkylamido C₅-C₂₀ aryl;
R⁵ is selected from the group consisting of hydrogen, hydroxyl, carboxyl, C₁-C₁₂ alkyl, C₁-C₁₂ alkoxy, C₂-C₁₂ alkylcarbonyl, C₂-C₁₂ carboxyalkyl, C₂-C₁₂ alkyloxycarbonyl, C₅-C₂₀ aryl, C₅-C₂₀ aryloxycarbonyl, C₅-C₂₀ aryl C₂-C₁₂ alkyloxycarbonyl, C₁-C₁₂ alkyl sulfonyl, C₁-C₁₂ hydroxyalkyl, formyl, C₂-C₁₂ formylalkyl, C₂-C₁₂ alkenyl, and C₂-C₁₂ alkynyl;
Ar is a C₅-C₂₀ monocyclic aryl group or a C₁₀-C₂₀ bicyclic aryl group or a heteroaryl group having 2 to 6 carbon atoms and one or more heteroatoms selected from the group consisting of N, O, S, and any combination thereof; and
bond "a" can be of α, β, or α/β configuration;
wherein any of R¹, R², R³, R⁴, and R⁵ other than hydrogen, halo, hydroxyl, nitro, cyanato, isocyanato, and thiocyanato may be further substituted with one or more substitutents selected from the group consisting of halo, hydroxyl, cyanato, isocyanato, thiocyanato, amino, C₁-C₁₂ alkyl, amido, nitro, and any combination thereof; or
a pharmaceutically acceptable salt or solvate thereof, for use in the treatment of a mental disorder.

18. The compound, salt or solvate of claim 17, wherein the mental disorder is selected from the group consisting of conduct disorders, preferably Attention Deficit Hyperactivity Disorder (ADHD), alcohol addiction, tobacco addiction, nicotine addiction, drug addiction, preferably cocaine abuse, sleep disorders, preferably narcolepsy, inhalation disorders, Parkinsonism including Parkinson's disease, female and male orgasmic disorders, female and male sexual arousal disorders, hypoactive sexual desire disorder, and anxiety and/or depression disorders.

19. A pharmaceutical composition comprising a compound, salt or solvate of any one of claims 1-16 and a pharmaceutically acceptable carrier.

20. A compound as defined in any one of claims 1-17 for use in selectively imaging cocaine binding sites of the central nervous system of a patient.

21. A compound as defined in any one of claims 1-17 for use in detecting or monitoring parkinsonism in a patient.

22. The compound, salt or solvate of any one of claims 1-16 for use in medicine.

23. Use of a compound, salt or solvate as defined in any one of claims 1-17 in the manufacture of a medicament for treating a mental disorder.

24. The use of claim 23, wherein the mental disorder is selected from the group consisting of conduct disorders, preferably Attention Deficit Hyperactivity Disorder (ADHD), alcohol addiction, tobacco addiction, nicotine addiction, drug addiction, preferably cocaine abuse, sleep disorders, preferably narcolepsy, inhalation disorders, Parkinsonism including Parkinson's disease, female and male orgasmic disorders, female and male sexual arousal disorders, hypoactive sexual desire disorder, and anxiety and/or depression disorders.

## Patentansprüche

1. Verbindung der Formel I: 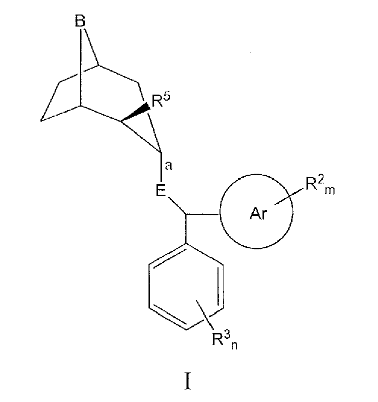 worin:
E NR¹, S oder CH₂ ist;
B NR⁴, O oder CH₂ ist;
R¹ ausgewählt ist aus der Gruppe bestehend aus Wasserstoff, C₁-C₁₂-Alkyl, C₁-C₁₂-Alkylamido-C₁-C₁₂-alkyl, C₁-C₁₂-Alkylamido-C₅-C₂₀-aryl, C₂-C₁₂-Alkylcarbonyloxy, C₂-C₁₂-Alkoxyalkyl, C₁-C₁₂-Hydroxyalkyl, C₃-C₁₂-Alkylcarbonyloxyalkyl, C₅-C₂₀-Aryl-C₁-C₁₂-alkyl, C₅-C₂₀-Aryloxy-C₁-C₁₂ -alkyl, Cinnamyl und C₂-C₁₂-Alkylcarbonyl;
m = 1 bis 5; n = 1 bis 3;
R² und R³ je unabhängig voneinander ausgewählt sind aus der Gruppe bestehend aus Wasserstoff, Halogen, C₁-C₁₂-Alkyl, C₁-C₁₂-Alkoxy, Nitro, Cyanato, Isocyanato, Thiocyanato, Amino, Halogen-C₁-C₁₂-alkyl, Hydroxyl, Trihalogen-C₁-C₁₂-alkyl und jeglicher Kombination aus diesen;
R⁴ ausgewählt ist aus der Gruppe bestehend aus Wasserstoff, C₂-C₁₂-Alkyl, C₂-C₁₂-Alkenyl, C₂-C₁₂-Alkynyl, C₅-C₂₀-Aryl-C₁-C₁₂-alkyl, C₅-C₂₀-Heteroaryl-C₁-C₁₂-alkyl, C₁-C₁₂-Alkylamino, Heterocyclyl-C₁-C₁₂-alkyl, C₁-C₁₂-Alkylsulfonyl, C₂-C₁₂-Alkylcarbonyl, (N(C₅-C₂₀-Aryl)amido)-C₁-C₁₂-alkyl, (N(C₁-C₁₂-Alkyl)amido)-C₁-C₁₂-alkyl, (N(C₅-C₂₀-Aryl)amido)-C₂-C₁₂-alkylcarbonyl, (N(C₁-C₁₂-Alkyl)amido)-C₂-C₁₂-alkylcarbonyl und C₁-C₁₂-Alkylamido-C₅-C₂₀-aryl;
R⁵ ausgewählt ist aus der Gruppe bestehend aus Wasserstoff, Hydroxyl, Carboxyl, C₁-C₁₂-Alkyl, C₁-C₁₂-Alkoxy, C₂-C₁₂-Alkylcarbonyl, C₂-C₁₂-Carboxyalkyl, C₂-C₁₂-Alkyloxycarbonyl, C₅-C₂₀-Aryl, C₅-C₂₀-Aryloxycarbonyl, C₅-C₂₀-Aryl-C₂-C₁₂-alkyloxycarbonyl, C₁-C₁₂-Alkylsulfonyl, C₁-C₁₂-Hydroxyalkyl, Formyl, C₂-C₁₂-Formylalkyl, C₂-C₁₂-Alkenyl und C₂-C₁₂-Alkynyl;
Ar eine monocyclische C₅-C₂₀-Arylgruppe oder eine bicyclische C₁₀-C₂₀-Arylgruppe oder eine Heteroarylgruppe mit 2 bis 6 Kohlenstoffatomen und einem oder mehreren Heteroatomen ausgewählt aus der Gruppe bestehend aus N, O, S und jeder Kombination aus diesen ist; und
die Bindung "a" von der α-, β- oder α/β-Konfiguration sein kann;
wobei jedes von R¹, R², R³, R⁴ und R⁵, das von Wasserstoff, Halogen, Hydroxyl, Nitro, Cyanato, Isocyanato und Thiocyanato verschieden ist, weiter substituiert sein kann mit einem oder mehreren Substituenten, die ausgewählt sind aus der Gruppe bestehend aus Halogen, Hydroxyl, Cyanato, Isocyanato, Thiocyanato, Amino, C₁-C₁₂-Alkyl, Amido, Nitro und jeder Kombination aus diesen; oder
ein pharmazeutisch annehmbares Salz oder Solvat davon;
mit der Massgabe dass:
Wenn die Bindung "a" von der β-Konfiguration ist, Ar Phenyl ist, R² Wasserstoff ist, B NCH₂CH₃ ist, E NH ist, R⁵ Wasserstoff ist und n = 1, R³ weder Wasserstoff noch p-Chloro ist.

2. Verbindung, Salz oder Solvat nach Anspruch 1, wobei E NR¹ ist.

3. Verbindung, Salz oder Solvat nach Anspruch 2, wobei R¹ Wasserstoff oder C₁-C₁₂-Alkyl, bevorzugt C₁-C₆-Alkyl ist.

4. Verbindung, Salz oder Solvat nach Anspruch 1 oder 2, wobei R³ Fluoro oder Chloro ist.

5. Verbindung, Salz oder Solvat nach einem der Ansprüche 1-4, wobei m = n = 1.

6. Verbindung, Salz oder Solvat nach einem der Ansprüche 1-5, wobei die Bindung "a" von der α-Konfiguration ist.

7. Verbindung, Salz oder Solvat nach einem der Ansprüche 1-6, wobei Ar ausgewählt ist aus der Gruppe bestehend aus Phenyl, Naphthyl, Biphenyl, Pyridyl, Bipyridyl, Pyrimidyl, Pyrrolyl, Furanyl, Thiophenyl, Triazolyl, Triazolopyrimidyl, Thiadiazolyl, Phosphol, Diazaphosphol, Chinoxalyl, Benzofuranyl, Benzopyrrolyl, Morpholinyl, Benzopyranyl, Oxolyl, Thiazolyl, Purinyl, Imidazolyl, Indolyl, Phosphindolyl, Pyrazolyl und Isoindolyl, und bevorzugt Phenyl ist.

8. Verbindung, Salz oder Solvat nach einem der Ansprüche 1-7, wobei R⁵ H oder C₂-C₁₂-Alkylcarbonyloxy, bevorzugt C₂-C₆-Alkylcarbonyloxy ist.

9. Verbindung, Salz oder Solvat nach einem der Ansprüche 1-8, wobei B NR⁴ ist.

10. Verbindung, Salz oder Solvat nach Anspruch 9, wobei R⁴ ausgewählt ist aus der Gruppe bestehend aus Wasserstoff, C₂-C₁₂-Alkyl, C₂-C₁₂-Alkenyl, C₅-C₂₀-Aryl-C₁-C₁₂-alkyl, C₁-C₁₂-Alkylamino, Heterocyclyl-C₁-C₁₂-Alkyl und (N(C₅-C₂₀-Aryl)amido)-C₁-C₁₂-Alkyl.

11. Verbindung, Salz oder Solvat nach Anspruch 2, wobei:
R¹ Wasserstoff oder C₁-C₁₂-Alkyl ist;
m=n= 1;
R² und R³ Halogen, bevorzugt Chloro oder Fluoro, oder Wasserstoff sind;
B NR⁴ ist;
R⁴ ausgewählt ist aus der Gruppe bestehend aus Wasserstoff, C₂-C₁₂-Alkyl, C₂-C₁₂-Alkenyl, C₅-C₂₀-Aryl-C₁-C₁₂-alkyl, C₁-C₁₂-Alkylamino, Heterocyclyl-C₁-C₁₂-Alkyl und (N(C₅-C₂₀-Aryl)amido)-C₁-C₁₂-alkyl;
R⁵ Wasserstoff ist;
Ar Phenyl ist; und
die Bindung "a" von der α-Konfiguration ist.

12. Verbindung, Salz oder Solvat nach Anspruch 2, wobei
R¹ Wasserstoff oder C₁-C₁₂-Alkyl ist;
m=n=2;
R² und R³ Halogen, bevorzugt Chloro oder Fluoro, oder Wasserstoff sind;
B NR⁴ ist;
R⁴ ausgewählt ist aus der Gruppe bestehend aus Wasserstoff, C₂-C₁₂-Alkyl, C₂-C₁₂-Alkenyl, C₅-C₂₀-Aryl-C₁-C₁₂-Alkyl, C₁-C₁₂-Alkylamino, Heterocyclyl-C₁-C₁₂-alkyl und (N(C₅-C₂₀-Aryl)amido)-C₁-C₁₂-alkyl,
R⁵ Wasserstoff ist;
Ar Phenyl ist; und
die Bindung "a" von der α-Konfiguration ist.

13. Verbindung nach Anspruch 1, wobei die Verbindung von der Formel II: 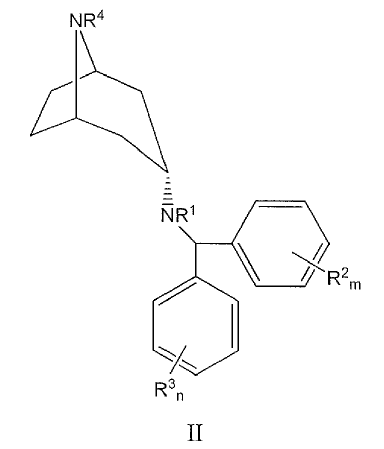 ist, worin:
R¹ Wasserstoff oder Methyl ist;
m = 1 oder 2 und n = 1 oder 2;
R² und R³ je unabhängig voneinander ausgewählt sind aus Wasserstoff, Fluoro und Chloro; und
R⁴ ausgewählt ist aus der Gruppe bestehend aus Wasserstoff, n-Butyl, Allyl, Phenylbutyl, 2-Ethylamino, [2-(1*H*-Indol-3-yl)-ethyl]- und 3-[(N-Phenyl)propionamido];
oder ein pharmazeutisch annehmbares Salz oder Solvat davon.

14. Verbindung, Salz oder Solvat nach Anspruch 13, wobei die Verbindung ausgewählt ist aus der Gruppe bestehend aus:

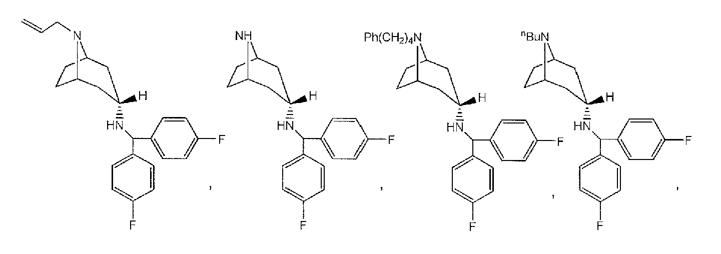

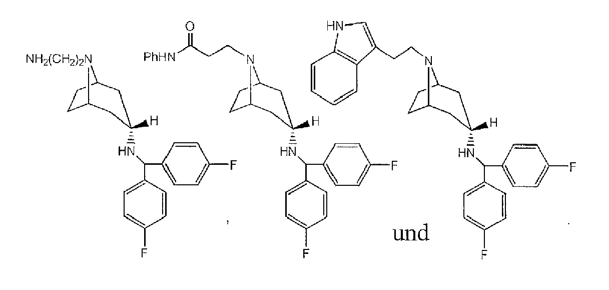

15. Verbindung, Salz oder Solvat nach Anspruch 1, wobei R⁴ C₂-C₁₂-Alkyl ist.

16. Verbindung ausgewählt aus der Gruppe bestehend aus: 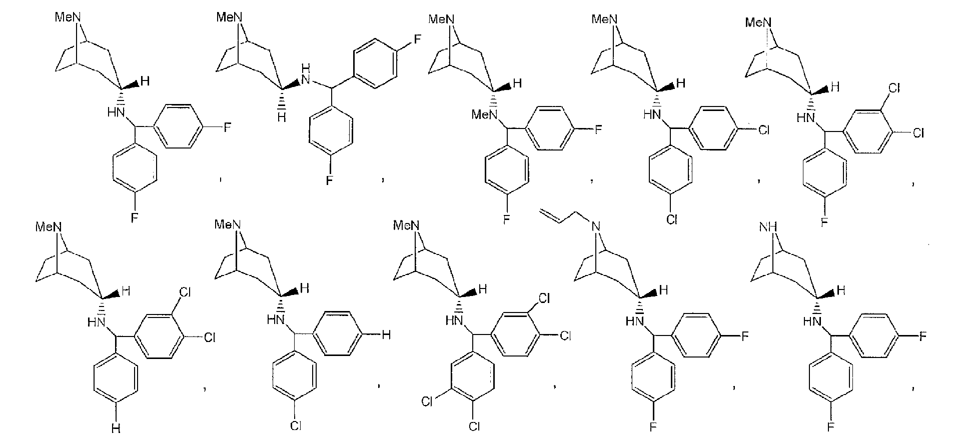 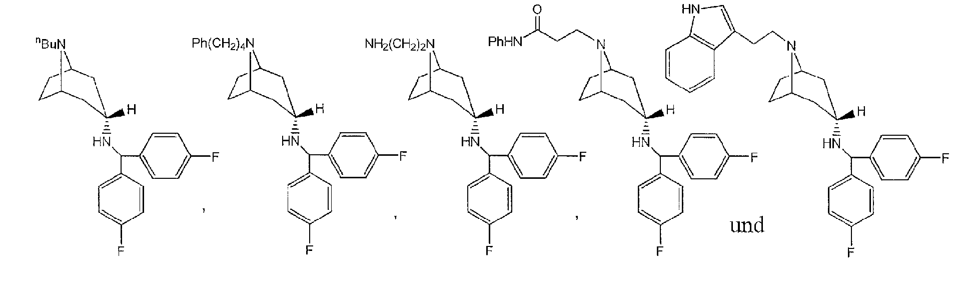 oder ein pharmazeutisch annehmbares Salz oder Solvat davon.

17. Verbindung der Formel I: 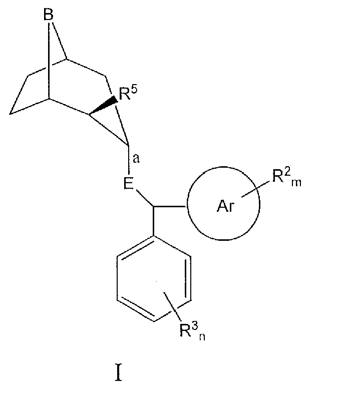 worin:
E NR¹, S oder CH₂ ist;
B NR⁴, O oder CH₂ ist;
R¹ ausgewählt ist aus der Gruppe bestehend aus Wasserstoff, C₁-C₁₂-Alkyl, C₁-C₁₂-Alkylamido-C₁-C₁₂-alkyl, C₁-C₁₂-Alkylamido-C₅-C₂₀-aryl, C₂-C₁₂-Alkylcarbonyloxy, C₂-C₁₂-Alkoxyalkyl, C₁-C₁₂-Hydroxyalkyl, C₃-C₁₂-Alkylcarbonyloxyalkyl, C₅-C₂₀-Aryl-C₁-C₁₂-alkyl, C₅-C₂₀-Aryloxy-C₁-C₁₂ -alkyl, Cinnamyl und C₂-C₁₂-Alkylcarbonyl,
m = 1 bis 5; n = 1 bis 3;
R² und R³ je unabhängig voneinander ausgewählt sind aus der Gruppe bestehend aus Wasserstoff, Halogen, C₁-C₁₂-Alkyl, C₁-C₁₂-Alkoxy, Nitro, Cyanato, Isocyanato, Thiocyanato, Amino, Halogen-C₁-C₁₂-alkyl, Hydroxyl, Trihalogen-C₁-C₁₂-alkyl und jeder Kombination aus diesen;
R⁴ ausgewählt ist aus der Gruppe bestehend aus Wasserstoff, C₁-C₁₂-Alkyl, C₂-C₁₂-Alkenyl, C₂-C₁₂-Alkynyl, C₅-C₂₀-Aryl-C₁-C₁₂-alkyl, C₅-C₂₀-Heteroaryl-C₁-C₁₂-alkyl, C₁-C₁₂-Alkylamino, Heterocyclyl-C₁-C₁₂-alkyl, C₁-C₁₂-Alkylsulfonyl, C₂-C₁₂-Alkylcarbonyl, (N(C₅-C₂₀-Aryl)ainido)-C₁-C₁₂-alkyl, (N(C₁-C₁₂Alkyl)amido)-C₁-C₁₂-alkyl, (N(C₅-C₂₀-Aryl)amido)-C₂-C₁₂-alkylcarbonyl, (N(C₁-C₁₂-Alkyl)amido)-C₂-C₁₂-alkylcarbonyl und C₁-C ₁₂-Alkylamido-C₅-C₂₀-aryl;
R⁵ ausgewählt ist aus der Gruppe bestehend aus Wasserstoff, Hydroxyl, Carboxyl, C₁-C₁₂-Alkyl, C₁-C₁₂-Alkoxy, C₂-C₁₂-Alkylcarbonyl, C₂-C₁₂-Carboxyalkyl, C₂-C₁₂-Alkyloxycarbonyl, C₅-C₂₀-Aryl, C₅-C₂₀-Aryloxycarbonyl, C₅-C₂₀-Aryl-C₂-C₁₂-alkyloxycarbonyl, C₁-C₁₂-Alkylsulfonyl, C₁-C₁₂-Hydroxyalkyl, Formyl, C₂-C₁₂-Formylalkyl, C₂-C₁₂-Alkenyl und C₂-C₁₂-Alkynyl,
Ar eine monocyclische C₅-C₂₀-Arylgruppe oder eine bicyclische C₁₀-C₂₀-Arylgruppe oder eine Heteroarylgruppe mit 2 bis 6 Kohlenstoffatomen und einem oder mehreren Heteroatomen ausgewählt aus der Gruppe bestehend aus N, O, S und jeder Kombination aus diesen ist; und
die Bindung "a" von der α-, β- oder α/β-Konfiguration sein kann;
wobei jedes von R¹, R², R³, R⁴ und R⁵, das von Wasserstoff, Halogen, Hydroxyl, Nitro, Cyanato, Isocyanato und Thiocyanato verschieden ist, weiter substituiert sein kann mit einem oder mehreren Substituenten, die ausgewählt sind aus der Gruppe bestehend aus Halogen, Hydroxyl, Cyanato, Isocyanato, Thiocyanato, Amino, C₁-C₁₂-Alkyl, Amido, Nitro und jeder Kombination aus diesen; oder
ein pharmazeutisch annehmbares Salz oder Solvat davon; zur Verwendung in der Behandlung einer mentalen Störung.

18. Verbindung, Salz oder Solvat nach Anspruch 17, wobei die mentale Störung ausgewählt ist aus der Gruppe bestehend aus Verhaltensstörungen, vorzugsweise Aufmerksamkeitsdefizit-/Hyperaktivitätsstörung (ADHD), Alkoholabhängigkeit, Tabakabhängigkeit, Nikotinabhängigkeit, Drogenabhängigkeit, vorzugsweise Kokainmissbrauch, Schlafstörungen, vorzugsweise Schlafkrankheit, Einatmungsstörungen, Parkinsonismus einschliesslich Parkinsonkrankheit, weibliche und männliche Orgasmusstörungen, weibliche und männliche sexuelle Erregungsstörungen, Störung mit verminderter sexueller Appetenz, und Angst- und/oder Depressionsstörungen.

19. Pharmazeutische Zusammensetzung umfassend eine Verbindung, ein Salz oder ein Solvat nach einem der Ansprüche 1-16 und einen pharmazeutisch annehmbaren Träger.

20. Verbindung wie in einem der Ansprüche 1-17 definiert, zur Verwendung in der selektiven Abbildung von Kokain-Bindungsstellen des Zentralnervensystems eines Patienten.

21. Verbindung wie in einem der Ansprüche 1-17 definiert, zur Verwendung in der Detektion oder der Überwachung von Parkinsonismus in einem Patienten.

22. Verbindung, Salz oder Solvat nach einem der Ansprüche 1-16 zur Verwendung in der Medizin.

23. Verwendung einer Verbindung, eines Salzes oder eines Solvates wie in einem der Ansprüche 1-17 definiert zur Herstellung eines Medikamentes zur Behandlung einer mentalen Störung.

24. Verwendung nach Anspruch 23, wobei die mentale Störung ausgewählt ist aus der Gruppe bestehend aus Verhaltensstörungen, vorzugsweise Aufmerksamkeitsdefizit-/Hyperaktivitätsstörung (ADHD), Alkoholabhängigkeit, Tabakabhängigkeit, Nikotinabhängigkeit, Drogenabhängigkeit, vorzugsweise Kokainmissbrauch, Schlafstörungen, vorzugsweise Schlafkrankheit, Einatmungsstörungen, Parkinsonismus einschliesslich Parkinsonkrankheit, weibliche und männliche Orgasmusstörungen, weibliche und männliche sexuelle Erregungsstörungen, Störung mit verminderter sexueller Appetenz, und Angst- und/oder Depressionsstörungen.

## Revendications

1. Composé de formule 1 : 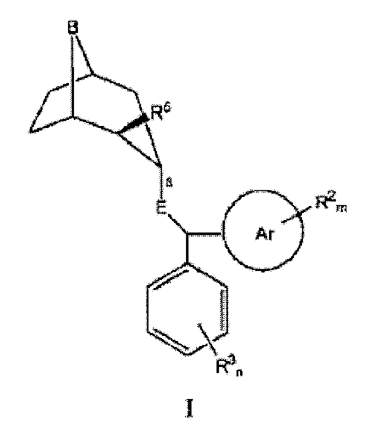 dans lequel :
E est un NR¹, S ou CH₂ ;
B est un NR⁴, 0 ou CH₂ ;
R¹ est choisi dans le groupe constitué d'hydrogène, alkyle en C₁-C₁₂, alkylamido en C₁-C₁₂ alkyle en C₁-C₁₂, alkylamido en C₁-C₁₂ aryle en C₅-C₂₀, alkylcarbonyloxy en C₂-C₁₂, alcoxyalkyle en C₂-C₁₂, hydroxyalkyle en C₁-C₁₂, alkylcarbonyloxyalkyle en C₃-C₁₂, aryle en C₅-C₂₀ alkyl en C₁-C₁₂, aryloxy en C₅-C₂₀ alkyle en C₁-C₁₂, cinnamyle et alkylcarbonyle en C₂-C₁₂ ;
m=1 à 5;n=1 à 3 ;
R² et R³ sont chacun indépendamment choisis dans le groupe constitué d'hydrogène, halo, alkyle en C₁-C₁₂, alcoxy en C₁-C₁₂, nitro, cyanato, isocyanato, thiocyanato, amino, halo alkyle en C₁-C₁₂, hydroxyle, trihaloalkyle en C₁-C₁₂ et toute combinaison de ceux-ci ;
R⁴ est choisi dans le groupe constitué d'hydrogène, alkyle en C₂-C₁₂, alcényle en C₂-C₁₂, alcynyle en C₂-C₁₂, aryle en C₅-C₂₀ alkyle en C₁-C₁₂, hétéroaryle en C₅-C₂₀ alkyle en C₁-C₁₂, alkylamino en C₁-C₁₂, hétérocyclylalkyle en C₁-C₁₂,alkylsulfonyle en C₁-C₁₂, alkylcarbonyle en C₂-C₁₂, (N(aryle en C₅-C₂₀)amido)-alkyle en C₁-C₁₂, (N(alkyle en C₁-C₁₂)amido)-alkyle en C₁-C₁₂, (N(aryle en C₅-C₂₀) amido)-alkylcarbonyle en C₂-C₁₂, (N(alkyle en C₁-C₁₂)amido)-alkylcarbonyle en C₂-C₁₂ et alkylamido en C₁-C₁₂ aryle en C₅-C₂₀;
R⁵ est choisi dans le groupe constitué d'hydrogène, hydroxyle, carboxyle, alkyle en C₁-C₁₂, alcoxy en C₁-C₁₂, alkylcarbonyle en C₂-C₁₂, carboxyalkyle en C₂-C₁₂, alkyloxycarbonyle C₂-C₁₂ aryle en C₅-C₂₀, aryloxycarbonyle en C₅-C₂₀, aryle en C₅-C₂₀ alkyloxycarbonyle en C₂-C₁₂, alkylsulfonyle en C₁-C₁₂, hydroxyalkyle en C₁-C₁₂, formyle, formylalkyle en C₂-C₁₂, alcényle en C₂-C₁₂ et alcynyle en C₂-C₁₂ ;
Ar est un groupe aryle monocyclique en C₅-C₂₀ ou un groupe aryle bicyclique en C₁₀-C₂₀ ou un groupe hétéroaryle ayant 2 à 6 atomes de carbone et un ou plusieurs hétéroatomes choisis dans le groupe constitué de N, 0, S et toute combinaison de ceux-ci ; et
la liaison "a" peut être de configuration α, β ou α/β ;
où quelconque de R¹, R², R³, R⁴ et R⁵ étant autre que hydrogène, halo, hydroxyle, nitro, cyanato, isocyanato, et thiocyanato peut être ultérieurement substitué par un ou plusieurs substituants choisis dans le groupe constitué de halo, hydroxyle, cyanato, isocyanato, thiocyanato, amino, alkyle en C₁-C₁₂, amido, nitro, et toute combinaison de ceux-ci ; ou
un sel ou solvate pharmaceutiquement acceptable de celui-ci;
sous réserve que ;
si la liaison "a" est de configuration β, Ar est phényle, R² est hydrogène, B est NCH₂CH₃, E est NH, R⁵ est hydrogène et n = 1, R³ n'est pas hydrogène ou p-chloro.

2. Composé, sel ou solvate selon la revendication 1, dans lequel E est un NR¹.

3. Composé, sel ou solvate selon la revendication 2, dans lequel R¹ est hydrogène, ou un alkyle en C₁-C₁₂, de préférence un alkyle en C₁-C₆.

4. Composé, sel ou solvate selon la revendication 1 ou 2, dans lequel R³ est fluoro ou chloro.

5. Composé, sel ou solvate selon l'une quelconque des revendications 1-4, dans lequel m = n = 1.

6. Composé, sel ou solvate selon l'une quelconque des revendications 1-5, dans lequel la liaison "a" est de configuration α.

7. Composé, sel ou solvate selon l'une quelconque des revendications 1-6, dans lequel Ar est choisi dans le groupe constitué de phényle, naphthyle, biphényle, pyridyle, bipyridyle, pyrimidyle, pyrrolyle, furanyle, thiophényle, triazolyle, triazolopyrimidyle, thiadiazolyle, phosphole, diazaphosphole, quinoxalyle, benzofuranyle, benzopyrrolyle, morpholinyle, benzopyranyle, oxolyle, thiazolyle, purinyle, imidazolyle, indolyle, phosphindolyle, pyrazolyle et isoindolyle, de préférence phényle.

8. Composé, sel ou solvate selon l'une quelconque des revendications 1-7, dans lequel R⁵ est H ou un alkylcarbonyloxy en C₂-C₁₂, de préférence un alkylcarbonyloxy en C₂-C₆.

9. Composé, sel ou solvate selon l'une quelconque des revendications 1-8, dans lequel B est un NR⁴.

10. Composé, sel ou solvate selon la revendication 9, dans lequel R⁴ est choisi dans le groupe constitué d'hydrogène, alkyle en C₂-C₁₂, alcényle en C₂-C₁₂, aryle en C₅-C₂₀ alkyle en C₁-C₁₂, alkylamino en C₁-C₁₂, hétérocyclylalkyle en C₁-C₁₂ et (N(aryle en C₅-C₂₀)amido)-alkyle en C₁-C₁₂.

11. Composé, sel ou solvate selon la revendication 2, dans lequel :
R¹ est hydrogène ou un alkyle en C₁-C₁₂ ;
m=n=1 ;
R² et R³ sont un halo, de préférence chloro ou fluoro, ou hydrogène ;
R est un NR⁴;
R⁴ est choisi dans le groupe constitué d'hydrogène, alkyle en C₂-C₁₂, alcényle en C₂-C₁₂, aryle en C₅-C₂₀-alkyle en C₁-C₁₂, alkylamino en C₁-C₁₂, hétérocyclylalkyle en C₁-C₁₂ et (N(aryle en C₅-C₂₀)amido)-alkyle en C₁-C₁₂ ;
R⁵ est hydrogène ;
Ar est phényle ; et
la liaison "a" est de configuration α.

12. Composé, sel ou solvate selon la revendication 2, dans lequel
R¹ est hydrogène ou un alkyle en C₁-C₁₂ ;
m = n = 2 ;
R² et R³ sont un halo, de préférence chloro ou fluoro, ou hydrogène ;
B est un NR⁴ ;
R⁴ est choisi dans le groupe constitué d'hydrogène, alkyle en C₂-C₁₂, alcényle en C₂-C₁₂, aryle en C₅-C₂₀ alkyle en C₁-C₁₂, alkylamino en C₁-C₁₂, hétérocyclylalkyle en C₁-C₁₂, et (N(aryle en C₅-C₂₀)amido)-alkyle en C₁-C₁₂ ;
R⁵ est un hydrogène ;
Ar est phényle ; et
la liaison "a" est de configuration α.

13. Composé selon la revendication 1, dans lequel le composé est de formule II : 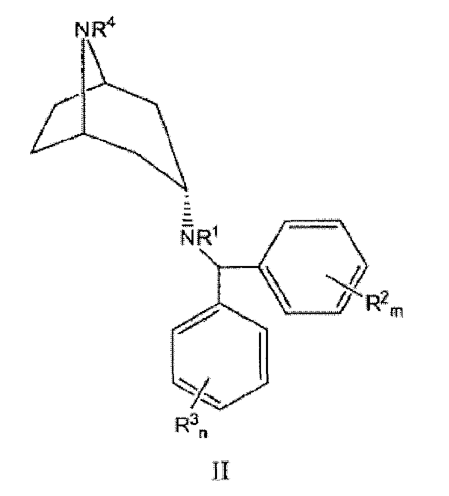 dans lequel :
R¹ est hydrogène ou méthyle ;
m = 1 ou 2 et n = 1 ou 2 ;
R² et R³ sont chacun indépendamment choisis parmi hydrogène, fluoro et chloro ; et
R⁴ est choisi dans le groupe constitué d'hydrogène, n-butyle, allyle, phénylbutyle, 2-éthylamino, [2-(1H-indol-3-yl)-éthyl]-, et 3-[(N-phényl) propionamido] ;
ou un sel ou solvate pharmaceutiquement acceptable de celui-ci.

14. Composé, sel ou solvate selon la revendication 13, dans lequel le composé est choisi dans le groupe constitué de :

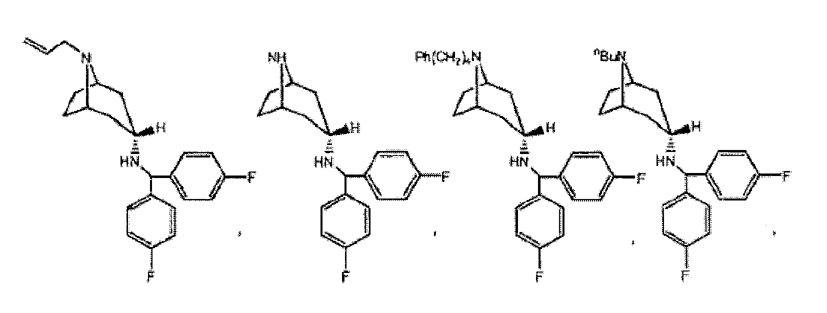

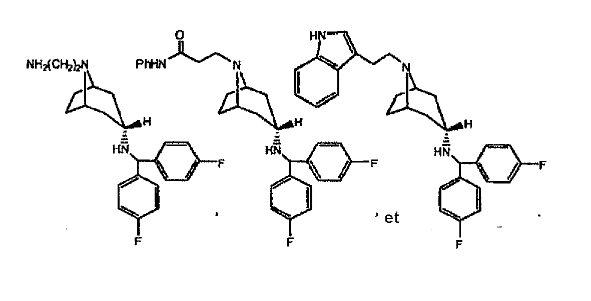

15. Composé, sel ou solvate selon la revendication 1, dans lequel R⁴ est un alkyle en C₂-C₁₂.

16. Composé choisi dans le groupe constitué de : 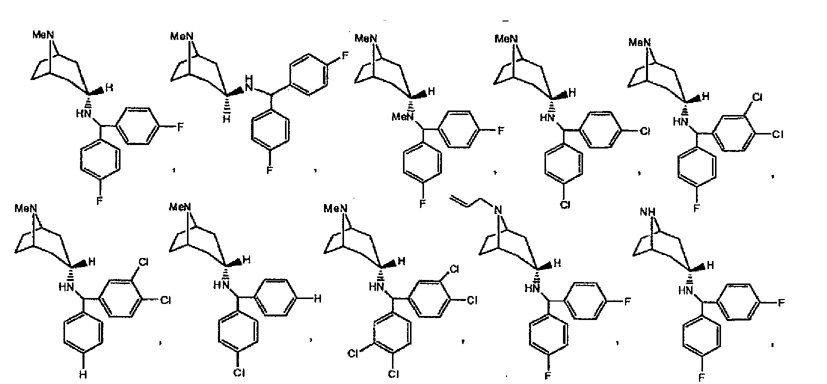 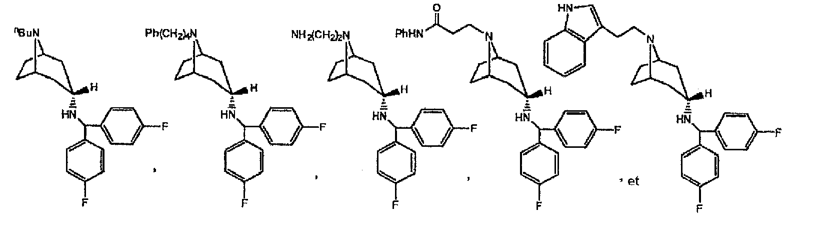 ou un sel ou solvate pharmaceutiquement acceptable de celui-ci.

17. Composé de formule I : 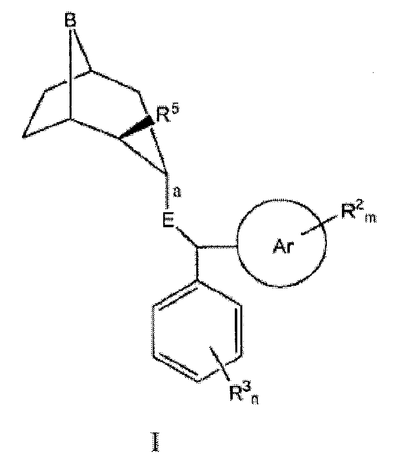 dans lequel :
E est un NR¹, S ou CH₂ ;
B est un NR⁴, O ou CH₂ ;
R¹ est choisi dans le groupe constitué d'hydrogène, alkyle en C₁-C₁₂, alkylamido en C₁-C₁₂ alkyle en C₁-C₁₂, alkylamido en C₁-C₁₂ aryle en C₅-C₂₀, alkylcarbonyloxy en C₂-C₁₂, alcoxyalkyle en C₂-C₁₂, hydroxyalkyle en C₁-C₁₂, alkylcarbonyloxyalkyle en C₃-C₁₂, aryle en C₅-C₂₀ alkyle en C₁-C₁₂, aryloxy en C₅-C₂₀ alkyle en C₁-C₁₂, cinnamyle, et alkylcarbonyle en C₂-C₁₂ ;
m = 1 à 5 ; n=1 à 3 ;
R² et R³ sont chacun indépendamment choisis dans le groupe constitué d'hydrogène, halo, alkyle en C₁-C₁₂, alcoxy en C₁-C₁₂, nitro, cyanato, isocyanato, thiocyanato, amino, haloalkyle en C₁-C₁₂, hydroxyle, trihaloalkyle en C₁-C₁₂ , et toute combinaison de ceux-ci ;
R⁴ est choisi dans le groupe constitué d'hydrogène, alkyle en C₁-C₁₂, alcényle en C₂-C₁₂ ; alcynyle en C₂-C₁₂, aryle en C₅-C₂₀ alkyle en C₁-C₁₂, hétéroaryl en C₅-C₂₀ alkyle en C₁-C₁₂, alkylamino en C₁-C₁₂, hétérocyclylalkyle en C₁-C₁₂, alkylsulfonyle en C₁-C₁₂, alkylcarbonyle en C₂-C₁₂, (N(aryle en C₅-C₂₀) amido)-alkyle en C₁-C₁₂, (N(alkyle en C₁-C₁₂)amido) alkyle en C₁-C₁₂, (N(aryle en C₅-C₂₀)amido)-alkylcarbonyle en C₂-C₁₂, (N(alkyle en C₁-C₁₂)amido)-alkylcarbonyle en C₂-C₁₂ et alkylamido en C₁-C₁₂ aryle en C₅-C₂₀.
R⁵ est choisi dans le groupe constitué d'hydrogène, hydroxyle, carboxyle, alkyle en C₁-C₁₂, alcoxy en C₁-C₁₂, alkylcarbonyle en C₂-C₁₂, carboxyalkyle en C₂-C₁₂, alcoxycarbonyle en C₂-C₁₂, aryle en C₅-C₂₀, aryloxycarbonyle en C₅-C₂₀, aryle en C₅-C₂₀ alkyloxycarbonyle en C₂-C₁₂, alkylsulfonyle en C₁-C₁₂, hydroxyalkyle en C₁-C₁₂, formyle, formylalkyle en C₂-C₁₂, alcényle en C₂-C₁₂ et alcynyle en C₂-C₁₂ ;
Ar est un groupe aryle monocyclique en C₅-C₂₀ ou un groupe aryle bicyclique en C₁₀-C₂₀ ou un groupe hétéroaryle ayant 2 à 6 atomes de carbone et un ou plusieurs hétéroatomes choisis dans le groupe constitué de N, 0, S et toute combinaison de ceux-ci ; et
la liaison "a" peut être de configuration α, β ou α/β ;
où quelconque de R¹, R², R³, R⁴ et R⁵ autre que hydrogène, halo, hydroxyle, nitro, cyanato, isocyanato, et thiocyanato peut être ultérieurement substitué par un ou plusieurs substituants choisis dans le groupe constitué de halo, hydroxyle, cyanato, isocyanato, thiocyanato, amino, alkyle en C₁-C₁₂, amido, nitro, et toute combinaison de ceux-ci ; ou
un sel ou solvate pharmaceutiquement acceptable de celui-ci, pour l'utilisation dans le traitement d'un trouble mental.

18. Composé, sel ou solvate selon la revendication 17, dans lequel le trouble mental est choisi dans le groupe constitué de troubles du comportement, de préférence un trouble d'hyperactivité et de déficit de l'attention (ADHD), l'alcoolisme, l'addiction au tabac, l'addiction à la nicotine, la toxicomanie, de préférence l'abus de cocaïne, les troubles du sommeil, de préférence la narcolepsie, les troubles de l'inhalation, le parkinsonisme, y compris la maladie de Parkinson, les troubles orgasmiques chez les femmes et les hommes, les troubles de la stimulation sexuelle chez les femmes et les hommes, les troubles du désir sexuel hypo-actif, et des troubles de l'anxiété et/ou de la dépression.

19. Composition pharmaceutique comprenant un composé, sel ou solvate selon l'une quelconque des revendications 1-16 et un support pharmaceutiquement acceptable.

20. Composé selon l'une quelconque des revendications 1-17, pour l'utilisation dans l'imagerie sélective des sites de liaison de la cocaïne du système nerveux central d'un patient.

21. Composé selon l'une quelconque des revendications 1-17, à utiliser dans la détection ou le contrôle du parkinsonisme chez un patient.

22. Composé, sel ou solvate selon l'une quelconque des revendications 1-16, pour l'utilisation dans la médicine.

23. Utilisation d'un composé, sel ou solvate selon l'une quelconque des revendications 1-17, dans la fabrication d'un médicament destiné à traiter un trouble mental.

24. Utilisation selon la revendication 23, dans laquelle le trouble mental est choisi dans le groupe constitué de troubles du comportement, de préférence un trouble d'hyperactivité et de déficit de l'attention (ADHD), l'alcoolisme, l'addiction au tabac, l'addiction à la nicotine, la toxicomanie, de préférence l'abus de cocaïne, les troubles du sommeil, de préférence la narcolepsie, les troubles de l'inhalation, le parkinsonisme, y compris la maladie de Parkinson, les troubles orgasmiques chez les femmes et les hommes, les troubles de la stimulation sexuelle chez les femmes et les hommes, les troubles du désir sexuel hypo-actif, et des troubles de l'anxiété et/ou de la dépression.
